(19) Europäisches Patentamt
European Patent Office
Office européen des brevets

(11) EP 0 878 996 B2

(12) **NOUVEAU FASCICULE DE BREVET EUROPEEN**

(45) Date de publication et mention de la décision concernant l'opposition:
**16.01.2008 Bulletin 2008/03**

(45) Mention de la délivrance du brevet:
**29.10.2003 Bulletin 2003/44**

(21) Numéro de dépôt: **97903430.3**

(22) Date de dépôt: **07.02.1997**

(51) Int Cl.:
***A21D 8/04*** *(2006.01)* ***C12N 1/18*** *(2006.01)*
*C12N 1/18* *(2006.01)* *C12R 1/865* *(2006.01)*

(86) Numéro de dépôt international:
**PCT/FR1997/000254**

(87) Numéro de publication internationale:
**WO 1997/028693 (14.08.1997 Gazette 1997/35)**

(54) **NOUVELLES LEVURES DE PANIFICATION SENSIBLES AU FROID**

NEUE KÄLTEEMPFINDLICHE BÄCKERHEFE

NOVEL COLD-SENSITIVE BREAD YEASTS

(84) Etats contractants désignés:
**AT BE CH DE DK ES FR GB IT LI NL SE**

(30) Priorité: **08.02.1996 FR 9601562**

(43) Date de publication de la demande:
**25.11.1998 Bulletin 1998/48**

(73) Titulaire: **LESAFFRE et Cie**
**F-75001 Paris (FR)**

(72) Inventeurs:
- **WADOUX, Isabelle**
**F-59700 Marcq-en-Baroeul (FR)**
- **COLAVIZZA, Didier**
**F-59163 Conde-sur-l'Escaut (FR)**
- **LOIEZ, Annie**
**F-59800 Lille (FR)**

(74) Mandataire: **Koch, Gustave**
**Cabinet Plasseraud**
**52 rue de la Victoire**
**75440 Paris Cedex 09 (FR)**

(56) Documents cités:
**EP-A- 0 487 878** **EP-A- 0 667 099**

EP 0 878 996 B2

## Description

La présente invention a pour objet des nouvelles souches de levures sensibles au froid et les procédés d'utilisation de ces souches en panification.

Les mutants de Saccharomyces cerevisiae sensibles au froid (en anglais "cold sensitive"), c'est-à-dire les mutants sensibles à des températures basses positives entre 0 et 15°C, sont abondamment décrits depuis longtemps dans la littérature scientifique. Les sites de mutation correspondants sont divers et de nombreux phénotypes différents peuvent être obtenus. La mutation peut affecter la croissance au froid, ou la fermentation au froid ou les deux, cette absence de croissance et/ou de fermentation peut être différente selon les sucres présents, elle peut être ou non réversible, c'est-à-dire disparaître ou ne pas disparaître quand la température augmente. L'emploi en panification de mutants, dont la fermentation des sucres des pâtes est limitée au froid mais rétablie à au moins 20°C, a essentiellement été étudié dans le but d'obtenir des pâtes pour croissants ou pour viennoiseries, ou encore des pâtes pour fonds de pizza destinées à être mises en oeuvre par la ménagère comme produit frais, c'est-à-dire après stockage à températures réfrigérées, en principe à + 4°C, mais en pratique entre 0 et + 12°C voire plus, dans le circuit de distribution, puis jusqu'à sa mise en oeuvre. De préférence ces pâtes réfrigérées destinées à la ménagère sont prêtes à cuire. On peut citer à cet égard les deux demandes de brevet européen EP 487878 et EP 663441 et les demandes de brevet internationales WO 93/01724 et WO 94/19955.

Dans les deux premiers documents cités, les documents EP 487878 et EP 663441, où une grande attention est portée au blocage de la fermentation du maltose, la quasi-totalité des mesures de dégagement de $CO_2$ à différentes températures en fonction du temps sont effectuées sur milieu synthétique maltosé où le maltose est le seul sucre fermentescible. L'application principale décrite est une pâte à pizza pouvant se conserver à températures réfrigérées, et qui en raison de cette conservation est prête à cuire pour la ménagère.

Dans les demandes de brevet internationales WO 93/01724 et WO 94/19955, de nombreux moyens sont décrits pour obtenir des pâtes destinées à la ménagère et se conservant longtemps à basse température. L'emploi de mutante dits lts (low temperature sensitive : sensible à basse température) disponibles dans les centres de collections universitaires comme le Yeast Genetic Stock Center du laboratoire Donner dans le Département de Biologie cellulaire et moléculaire de l'Université de Californie à Berkeley est l'une des options étudiées, la solution préférée étant semble-t-il l'emploi de souches incapables de fermenter le glucose, mais pouvant fermenter soit le fructose, soit le galactose, le sucre destiné à être fermenté étant juste présent dans la quantité nécessaire pour assurer la levée désirée de la pâte.

La demande de brevet européen EP 667099 décrit l'obtention et l'emploi en panification d'un mutant sensible au froid obtenu à partir d'une souche commerciale de levure de boulangerie classique pour pâtes sucrées, c'est-à-dire d'une souche lente non adaptée au maltose. On peut également citer pour mémoire la demande de brevet EP 556905 qui se borne à décrire différents protocoles opératoires susceptibles de permettre en théorie l'obtention de mutants sensibles au froid.

La demande de brevet européen EP-A-0 818 535, considérée comprise dans l'état de la technique en vertu de l'Article 54(3) CBE, décrit un gène correspondant au gêne YLR087c de Saccharomyces cerevisiae codant pour une protéine qui est capable de complémenter la mutation correspondant à une fermentation des levures sensible aux basses températures, des levures construites avec une inactivation de ce gène, ainsi que des pâtes réalisées avec lesdites levures.

Cette abondante littérature a donné lieu à très peu d'applications pratiques. On peut seulement citer deux exemples :

- la commercialisation en Europe d'une pâte à pizza se conservant longtemps au froid et prête à cuire après cette conservation au froid ;
- la commercialisation au Japon à prix très élevé et de manière assez limitée d'une levure pour pâtes sucrées dont l'activité fermentative est ralentie à basse température.

On peut remarquer que la fabrication de la pâte à pizza est une fabrication pour laquelle il est généralement souhaité des levures peu actives au point de vue dégagement gazeux, la levure ayant d'abord un rôle d'apport d'arômes. On peut remarquer que la souche commercialisée au Japon est une souche lente non adaptée au maltose, alors que les souches les plus utilisées pour la production de levures de panification dans le monde sont des souches rapides adaptées au maltose.

L'invention a pour objet des nouvelles souches obtenues par des méthodes classiques de mutation et présentant des progrès par rapport à cet état de la technique, les levures de panification fraîches et sèches obtenues avec ces souches, l'application de ces souches dans différents procédés de fabrication de pains ou d'autres produits de panification, notamment de pains de type français.

L'invention a également pour objet les nouvelles souches obtenues par mutagenèse dirigée, effectuée par biologie moléculaire, consistant à reproduire spécifiquement dans les souches industrielles de levures de panification, ou dans les haploïdes de départ ayant servi à la construction desdites souches industrielles, les mutations, monogéniques ou non, donnant le phénotype recherché dans les souches sélectionnées après traitement de mutation classique par exemple aux agents chimiques. Une variante, selon l'invention, de construction de souches ayant leur fermentation bloquée à basse température, et rétablie au-dessus de 20°C, est la transformation de souches industrielles de levure de panification avec un gène choisi pour avoir une action directe ou indirecte sur la fermentation des sucres et dont l'expression est conditionnée par la température.

L'invention a notamment pour objet une nouvelle souche de levure de panification donnant des levures fraîches ou sèches pouvant être qualifiées de levures rapides à 30°C sur pâte sans ajout de sucre, c'est-à-dire des levures fraîches donnant au moins 100 ml de $CO_2$ dans le test $A_1$ en 2 heures à 30°C, de préférence au moins 110 ml de $CO_2$ et de préférence encore au moins 150 ml. Ces nouvelles levures fraîches et sèches obéissent de plus au rapport suivant :

$$\frac{\text{dégagement } CO_2 \text{ en 48 heures à 8°C dans le test } A_1}{\text{dégagement } CO_2 \text{ en 2 heures à 30°C dans le test } A_1} =$$

inférieur à 45% et de préférence inférieur à 40% et encore de préférence inférieur à 30%. De préférence, ces nouvelles levures fraîches et sèches obéissent aux mêmes pourcentages, si ce rapport est mesuré avec le test $A_5$, et de préférence avec ce test $A_5$ ce rapport sera inférieur ou égal à 20%.

L'invention a également pour objet l'utilisation des nouvelles souches obtenues dans les procédés de panification différée de production de pains, notamment de pains de type français ou de pains à faible teneur en sucre, c'est-à-dire à moins de 5% de sucre.

Un procédé de panification différée est défini comme tout procédé où il y a plus de 6 heures entre le pétrissage et la cuisson, et généralement plus de 12 heures. Notamment, l'utilisation de levures fraîches ou sèches obtenues avec une souche de levure de panification sensible au froid apporte un progrès important dans tous les procédés de panification différée comme les procédés de pousse lente ou de pousse bloquée ci-après définis; cette utilisation permet de conduire en toute sécurité à des pâtons qui restent prêts à cuire pendant une période d'au moins 4 heures, et de préférence au moins 8 heures, en donnant des produits cuits sans défaut.

L'invention a enfin pour objet des nouveaux procédés de panification, comme des procédés d'élaboration de levains ou l'utilisation de pâtes en vrac, basés sur l'emploi des levures de panification sensibles au froid.

Les tests utilisés par la Demanderesse pour caractériser les susdites levures sont réalisés à l'aide du fermentomètre de Burrows et Harrison décrit dans le "Journal of the Institute of Brewing", vol. LXV, No.1, January-February 1959 et sont exactement définis de la manière suivante:

Test $A_1$ (levures comprimées fraîches).

A 20 g de farine incubée à la température choisie pour la mesure, on ajoute un poids de levure comprimée correspondant à 160 mg de matières sèches, cette levure étant délayée dans 15 ml d'eau contenant 27 g de NaCl par litre et 4 g de $(NH_4)_2SO_4$ par litre; on malaxe à l'aide d'une spatule pendant 40 secondes, de manière à obtenir une pâte que l'on place au bain-marie réglé à la température choisie; treize minutes après le début du malaxage, le récipient contenant la pâte est fermé hermétiquement; la quantité totale de gaz produit est mesurée après 60, puis 120 minutes ou plusieurs heures; cette quantité est exprimée en ml à 20°C et sous 760 mm de Hg.

Pour toutes les levures susceptibles de donner en 120 minutes un dégagement gazeux égal ou supérieur à 150 ml de $CO_2$, la quantité de sucres fermentescibles apportée uniquement par la farine est limitante; en conséquence, le test est modifié de la manière suivante: on ajoute un poids de levure correspondant à 106 mg de matières sèches levure, au lieu de 160 mg, et la lecture de la quantité de gaz produite est par convention multipliée par 1,5.

Test $A'_1$ (levures sèches).

Identique à l'essai $A_1$, mais préalablement au malaxage, on réhydrate en 15 minutes les 160 mg de matières sèches levure qui se présentent sous forme de levure sèche active dans de l'eau distillée, à 38°C, on utilise à cet effet 40% du volume d'eau d'hydratation mis en oeuvre; le complément en eau, additionné de 405 mg de NaCl, est ajouté à l'issue des 15 minutes de réhydratation.

Test $A_3$ (levures comprimées fraîches).
Essai identique à l'essai $A_1$, mais on ajoute à la farine 2 g de saccharose; la quantité totale de gaz produit est mesurée après 60 minutes, 120 minutes ou plusieurs heures.
Test $A'_3$ (levures sèches).
Essai identique à l'essai $A'_1$, mais on ajoute à la farine 2 g de saccharose; la quantité totale de gaz produit est mesurée après 60 minutes, 120 minutes ou plusieurs heures.
Test $A_5$ (levures comprimées fraîches).
Essai identique à l'essai $A_1$, mais on ajoute à la farine 4 g de saccharose; la quantité totale de gaz produit est mesurée après 60 minutes et 120 minutes, ou plusieurs heures.
Test $A'_5$ (levures sèches).
Essai identique à l'essai $A'_1$, mais on ajoute à la farine 4 g de saccharose; la quantité totale de gaz produit est mesurée après 60 minutes et 120 minutes ou plusieurs heures.
Test $A_6$ (levures comprimées fraîches).
A 25 g de farine incubée à 30°C, on ajoute 6,5 g de sucre glace et un poids de levure comprimée correspondant à 320 mg de matières sèches, ensuite on procède comme pour l'essai $A_1$ et la quantité totale de gaz produit est mesurée après 60 et 120 minutes ou plusieurs heures.
Test $A'_6$ (levures sèches).
Essai identique à l'essai $A_6$, en procédant pour réhydrater les 320 mg de matières sèches levure sous forme levure sèche active comme dans l'essai $A'_1$.

Les milieux de culture utilisés sont les suivants:

| | |
|---|---|
| YPG | 20 g/litre de glucose, 20 g/litre de bactopeptone, 10 g/litre d'extrait de levure. |
| YPM | 20 g/litre de maltose, 20 g/litre de bactopeptone, 10 g/litre d'extrait de levure. |
| YPM/G | 20 g/litre de maltose, 2 g/litre de glucose, 20 g/litre de bactopeptone, 10 g/litre d'extrait de levure. |
| YEG-Agar | 20 g/litre de glucose, 5 g/litre d'extrait de levure, Agar 30 g/litre. |

Ceci étant, pour construire les nouvelles souches conformément à l'invention, on procède comme suit.

Dans une première étape, on sélectionne les souches parentes qui seront soumises au traitement de mutation. De préférence, on sélectionne les souches déjà connues pour être utilisées industriellement pour la production de levures de panification, ces souches ayant déjà les propriétés de base indispensables; la plupart de ces souches peuvent être obtenues auprès des centres de collection internationaux et notamment à l'ATCC (American Type Culture Collection), où elles sont déposées. Elles peuvent également être isolées à partir des levures de panification fraîches ou sèches du commerce.

Elles peuvent être classées en trois grands groupes:

- les souches rapides sur pâte sans sucre, c'est-à-dire bien adaptées au maltose qui ont des activités maltose perméase et maltase, constitutives et non réprimées par le glucose;
- les souches lentes bien adaptées aux pâtes sucrées, souches osmotolérantes qui ont des activités maltose perméase et/ou maltase après culture sur glucose faibles, voire très faibles;
- les souches construites pour réunir les propriétés des deux groupes précédents, comme celles décrites dans la demande de brevet européen n° 92 401168.7 du 23-04-1992 publiée sous le n° 0 511 108.

On sélectionne comme souches parentes, les meilleures souches de levures de boulangerie connues jusqu'alors dans ces différents groupes. Une souche sera en général d'autant moins active à basse température, qu'elle est peu active à 30°C, la température utilisée habituellement pour les mesures d'activité fermentative. Si dans l'application finale on n'a pas besoin d'une activité fermentative élevée, on a intérêt à partir de souches peu performantes, non adaptées au maltose pour rechercher après mutagenèse, les mutants dont la fermentation sera ralentie à basse température par rapport à la souche parente. C'est pour cela que l'art antérieur enseigne en général qu'il ne faut pas partir de souches rapides adaptées au maltose. Cependant, de telles souches ne peuvent intéresser que des créneaux très spécifiques. Selon la présente invention, les souches parentes soumises au traitement de mutagenèse seront de préférence des souches rapides bien adaptées au maltose. Ces souches, diploides ou polyploides, seront de manière générale des souches polyploides.

Ces souches diploïdes ou polyploides ainsi sélectionnées sont alors soumises à un traitement de mutagenèse classique, par exemple à l'aide d'agents chimiques. Les mutants obtenus sont incubés sur milieu classique glucose comme le milieu YPG, en conditions aérobies (fioles agitées), pendant quelques heures à 30°C, puis soumis à une première sélection par cultures strictement anaérobies sur milieu glucosé avec enrichissement à la nystatine ou tout agent connu comme tuant les cellules ayant une activité métabolique, ces cultures strictement anaérobies étant effectuées à une température choisie entre 10°C et 15°C. On étale ensuite la suspension cellulaire résultant de l'enrichissement à la nystatine ou tout agent similaire, à une dilution appropriée pour obtenir des colonies isolées des mutants qui n'ont pas été tués parce qu'ils n'ont pas d'activité métabolique à la température choisie entre 10 et 15°C. Des agents équivalents à la nystatine sont par exemple les dérivés du tritium décrits par LITTLEWOOD, B.S. and DAVIES, J.E. dans l'article "Enrichment for temperature sensitive and auxotrophic mutants in Saccharomyces cerevisiae by Tritium suicide", Mutation Research 1973, 17, 315-322.

Les souches diploides ou polyploïdes obtenues selon le procédé classique de mutagenèse décrit ci-dessus sont sélectionnées par mesure de leur consommation de différents sucres en anaérobiose à basse température.

Les différents sucres intervenant dans la fermentation panaire sont décrits ci-dessous. Dans les pâtes constituées uniquement de farine, d'eau, de sel et de levure (panification de type français), on retrouve en général :

- moins de 0,1% en poids par rapport à la farine d'hexoses simples directement fermentescibles, essentiellement sous forme de glucose;
- un peu moins ou environ 1% d'hexoses simples (glucose et fructose) libérés par l'action de l'invertase de la levure;
- quelques pour cent de maltose (de l'ordre de 2 à 3%) provenant de l'action des amylases présentes dans la pâte. Le maltose est le sucre principal, et il doit être fermenté en même temps, ou au moins sans retard après la consommation du fructose et du glucose libéré par l'invertase des levures. On remarquera qu'à moins de partir d'une souche de levure sans invertase, on aura toujours lors du démarrage de la fermentation panaire du glucose et du fructose présent en même temps que le maltose. Dans les pâtes où il y a ajout de sucres, celui-ci est soit du saccharose, du glucose, ou encore de l'isoglucose (mélange de glucose et de fructose). Le saccharose est inverti par l'invertase des levures en glucose et fructose.

Selon les tests de sélection utilisés pour réaliser la présente invention, une biomasse connue de mutants diploïdes ou polyploïdes sélectionnés comme décrit ci-dessus est testée en mesurant la consommation par cette biomasse de glucose (milieu glucosé) ou de maltose et glucose (milieu maltosé avec un peu de glucose) ou encore de maltose seul en anaérobiose à une température entraînant la sensibilisation au froid, de préférence, entre 8 et 15°C, pendant un temps tel qu'environ 90% des sucres présents dans le milieu sont consommés par la souche parente de départ. Les sucres résiduels sont dosés par le réactif au dinitrosalicylate de sodium.

Ce test de sélection peut être avantageusement réalisé de la manière suivante. Les mutants sélectionnés comme décrit ci-dessus, après culture dans des conditions tuant les cellules ayant un métabolisme actif en anaérobie à une température choisie entre 10 et 15°C, sont cultivés sur milieu YEG-AGAR pendant 24 heures. Les biomasses obtenues sont récoltées de manière à obtenir une suspension dans l'eau distillée de densité optique constante, par exemple 15, ce qui correspond à environ 7 mg de matières sèches de biomasse levure par millilitre. 10 microlitres de cette suspension de levure sont incubés en présence de 100 microlitres de milieu de culture sur plaques de microtitration, 1 plaque de microtitration permettant de faire le test sur au moins 90 mutants à la fois. Les plaques de microtitration sont ensuite incubées à la température choisie, pendant un temps tel que le ou les témoins consomment 90% des sucres témoins. Les sucres présents sont dosés par ajout de 100 μl par puits ou cupules du réactif au dinitrosalicylate de sodium à 1% après chauffage de la plaque de microtitration à 55°C pendant 30 min. La consommation des sucres de départ peut être mesurée:

- soit par une mesure directe de l'absorbance à 550 nanomètres par un lecteur de plaques de microtitration après transfert du milieu réactionnel dépourvu de levure dans une autre plaque de microtitration,
- soit par une mesure au spectrophotomètre à 550 nm après dilution au dixième du milieu de réaction.

Dans le cadre de cette mesure, on sélectionne les mutants qui sur le milieu YPM/G consomment moins de 60% du sucre consommé par le témoin.

Ces tests peuvent être faits également sur milieu YPG ou sur milieu YPM.

Cette procédure de sélection a permis entre autres d'obtenir les 3 souches suivantes qui ont été déposées au Centre National de Culture de Microorganismes (CNCM), Institut Pasteur Paris:

- la souche S47-12b1 déposée au CNCM sous le n° I-1645, le 05-12-1995,
- la souche L30-13 déposée au CNCM sous le n° I-1647, le 05-12-1995
- la souche L30-91 déposée au CNCM sous le n° I-1646, le 05-12-1995.

La souche S47-12b1 est un mutant d'une souche de levure rapide, adaptée au maltose, utilisée industriellement pour la production de levures de panification.

Cette souche S47-12b1 permet d'obtenir des levures fraîches pressées comparables aux levures fraîches pressées du type rapide du commerce lorsqu'elle est utilisée dans des conditions normales. Elle permet d'obtenir des levures fraîches pressées qui, lorsqu'elles sont cultivées de manière à contenir 7% d'azote sur matières sèches, donnent au moins 100 ml de $CO_2$ dans le test $A_1$ en 2 heures à 30°C et de préférence au moins 110 ml. et qui lorsqu'elles sont cultivées de manière à contenir 8,2% d'azote sur matières sèches, donnent au moins 150 ml de $CO_2$ dans le test $A_1$ en 2 heures à 30°C, et de préférence au moins 160 ml de $CO_2$. Cette souche permet d'obtenir des levures sèches donnant au moins 100 ml de $CO_2$ dans le test $A_1$ en 2 heures à 30°C. Ces levures fraîches ont de plus la propriété de respecter les rapports:

$$\frac{\text{dégagement } CO_2 \text{ en 48 heures à 8°C dans le test } A_1}{\text{dégagement } CO_2 \text{ en 2 heures à 30°C dans le test } A_1} \text{ de l'ordre de 40\%}$$

$$\frac{\text{dégagement } CO_2 \text{ en 2 heures à 30°C dans le test } A_5}{\text{dégagement } CO_2 \text{ en 48 heures à 8°C dans le test } A_5} \text{ de l'ordre de 20\%}$$

alors que ces rapports sont plus de 100% pour les levures fraîches obtenues avec la souche parente, levures fraîches qui sont en permanence disponibles dans le commerce, de manière très courante et usuelle, en Europe.

Ces rapports sont également vérifiés pour les levures sèches obtenues avec cette souche.

Les souches L30-13 et L30-91 déposées au CNCM respectivement sous les n° I-1647 et I-1646 sont des mutants d'une souche osmotolérante utilisée industriellement pour la production de levures de panification pour pâtes sucrées, commercialisées de manière usuelle sur le marché européen. Ces deux souches permettent d'obtenir des levures pressées fraîches ou des levures sèches comparables aux levures commerciales obtenues avec la souche parente. Ces levures ont de plus la propriété de respecter les rapports suivants:

| Rapport entre 48 heures de fermentation à 8°C/ 2 heures de fermentation à 30°C exprimé en % | | |
|---|---|---|
| | test $A_1$ | test $A_5$ |
| L30-13 | 20 % | 3 % |
| L30-91 | 5 % | 1 % |

alors que ces deux rapports sont plus de 100% pour les levures fraîches de panification obtenues avec la souche parente de départ.

La souche S47-12b1 est un mutant de souche rapide qui a gardé au-dessus de 20°C, toutes les propriétés de la souche "commerciale" dont elle est issue. Elle est particulièrement intéressante en panification différée de type français, c'est-à-dire en panification où il n'y a pas de sucre ou peu de sucre ajouté à la pâte.

Cette souche est particulièrement intéressante pour être utilisée dans un procédé de pousse contrôlée classique où la fermentation est bloquée après le façonnage des pâtes et avant la dernière fermentation ou apprêt. Ce blocage de la fermentation peut se faire à température plus élevée qu'habituellement, jusqu'à au moins 8°C ou 10°C ou à la même température qu'habituellement (entre -2°C et +4°C) mais pendant des temps plus longs qu'habituellement. Elle est aussi particulièrement intéressante dans les procédés dits de pousse bloquée ou de pousse lente. La pousse bloquée est un procédé où la fermentation est bloquée après l'apprêt, juste avant la mise au four. La souche S47-12b1 permet des blocages de 4 à 24 heures à environ 8°C (c'est-à-dire entre 4 et 10°C). La pousse lente est un procédé où c'est l'apprêt qui est très lent et réalisé à des températures entre 10 et 18°C, soit environ 15°C pendant 15 à 24 heures. Ces deux procédés sont intéressants car ils permettent une plage de mise au four très large, pour étaler la cuisson dans le temps, et proposer en permanence des pains chauds qui viennent d'être cuits, sans tous les inconvénients d'un recours à la congélation.

Les souches L30-13 et L30-91 sont particulièrement intéressantes pour les panifications de pâtes sucrées. Ces deux souches permettent de réaliser tous les schémas de panification différée des pâtes sucrées avec les avantages liés à leur phénotype de sensibilité au froid. La souche L30-91 est particulièrement intéressante pour toutes les pâtes destinées à la ménagère, du fait de son activité fermentative extrêmement faible à 8°C. Ces deux souches permettent notamment de préparer des pâtes pour viennoiserie notamment des croissants pour la ménagère, pouvant être conservés pendant 1 mois à 8°C, ces pâtes étant cuites après un apprêt d'une nuit à 20°C.

Les levures de panification obtenues avec ces souches sensibles au froid sont également intéressantes pour la réalisation de pâtons congelés, car elles permettent plus facilement de bloquer tout démarrage de la fermentation lors du pétrissage, de la division et du façonnage des pâtes, réalisés à basse température, étant entendu qu'il est bien connu que les métabolites issus de la fermentation panaire sont toxiques pour les cellules de levure présentes dans les pâtons congelés.

De manière générale, les levures de panification sensibles au froid peuvent apporter une aide importante au travail du boulanger. Elles peuvent permettre d'obtenir des pièces de panifications prêtes à cuire sur 4 à 8 heures. Elles peuvent permettre également des progrès ou le développement de nouveaux procédés au niveau de la préparation et de la conservation de pâtes ou de levains en masse (c'est-à-dire en vrac).

Les levures de panification sensibles au froid permettent de réaliser en masse des pâtes ou des levains qui peuvent, après avoir été amenés à une température en-dessous de 10°C, être stockés plusieurs jours si nécessaire puis transportés. Elles permettent de réaliser la préfermentation panaire correspondant au levain et/ou la première fermentation panaire, intervenant avant la division et/ou le façonnage, de manière découplée de l'ensemble de la fabrication panaire, et de manière regroupée, alors qu'il s'agit d'opérations demandant beaucoup de temps. Ces pâtes en masse ou en vrac peuvent être stockées en cuves ou bacs dans des chambres froides ou en seaux. Ces applications sont particulièrement intéressantes en panification française, mais elles ne sont pas limitées à cette panification. Elles permettent par exemple une fabrication et une distribution en seaux de pâtes jaunes fermentées comme des pâtes pour pancakes, ou des pâtes pour blinis qui à environ 4°C peuvent être distribuées à tous les points de cuisson.

Les levures sensibles au froid permettent de réaliser des levains liquides de type poolish, flour-brew, liquid sponge et de les conserver plusieurs jours avant emploi. Elles permettent également de la même manière de réaliser des levains pâteux, comme les levains-levures, les sponges. Tous ces levains peuvent servir à faire des pains, mais aussi des brioches, des doughnuts, des crackers.

Une application particulièrement intéressante est de réaliser avec ces levures sensibles au froid, et de préférence avec une levure sensible au froid rapide et adaptée au maltose des pâtes complètes ayant subi une première fermentation longue (en terme de métier un pointage long), de manière à obtenir avec ces pâtes ensuite en 2 à 3 heures maximum des pains très aromatiques. Cela permet notamment de revenir en pain courant français (pains obtenus par pétrissage uniquement de farine, eau, levure et éventuellement levain, sel, sans aucun ajout de sucre ou de matières grasses) à des procédés anciens avec des premières fermentations longues, et des apprêts courts. L'emploi d'une levure sensible au froid, et de préférence d'une levure rapide sensible au froid et adaptée au maltose permet d'avoir une masse de pâte en vrac ayant subi un pointage long disponible pour des fabrications rapides de pains français à l'ancienne.

Une application nouvelle des levures de boulangerie sensibles au froid est leur utilisation dans la régulation par la température de la croissance des différents micro-organismes dans un levain acide contenant des levures et des bactéries.

Si le levain panaire est ensemencé de manière à être une culture mixte d'un microorganisme sensible au froid à fort taux de croissance et d'un autre microorganisme psychrophile, on peut alors régler le rapport des deux populations en déterminant ou faisant varier la température de fermentation.

On peut ainsi orienter la production de biomasse, et donc des métabolites produits par la biomasse, et favoriser ainsi ou non la production de bactéries, et donc d'acides lactique et acétique.

Une variante de construction des nouvelles souches selon l'invention réside dans la construction desdites souches par les techniques de la biologie moléculaire, comme indiqué ci-dessous.

Une première étape peut avantageusement consister à mettre en évidence des différences de synthèse des protéines solubles et/ou des protéines membranaires des levures à 10°C et 30°C entre un mutant sélectionné, comme sensible au froid et la souche parente. Un mutant sélectionné sensible au froid est un mutant dont la fermentation est bloquée ou au moins fortement ralentie à des températures égales ou inférieures à 15°C et est normale au-dessus de 20°C, quand cette fermentation est mesurée par rapport à la souche parente de départ.

Ces comparaisons peuvent être faites par la technique d'électrophorèse bidimensionnelle des protéines avec comparaison des électrophorégrammes 2-D par un logiciel d'analyse approprié, comme le logiciel 2-D Analyzer®, commercialisé en France par BIOIMAGE, 777, E. Eisenhower Parkway, Suite 950, Ann Arbor, Michigan 48108, USA. Le but de ces comparaisons est de déceler des différences importantes dans la synthèse des protéines, et si ces dernières sont identifiables à partir des indexations gène-protéine connues, comme celles décrites dans l'article "Two-dimensional Protein Map of Saccharomyces cerevisiae : Construction of a Gene-Protein Index", ayant pour titre abrégé: "S. cerevisiae 2D protein map", de BOUCHERIE H. et al., publié dans "Yeast" 1995, 11(7), 601-613. Si la protéine n'est pas connue, il est possible dans certains cas de la recueillir sur le gel d'électrophorèse, de déterminer sa séquence $NH_2$-terminale, et de remonter au gène qui a été muté, d'autant plus que le génome de la levure est pratiquement aujourd'hui entièrement connu. La connaissance du ou des gènes objets de la mutation, permet alors par des techniques classiques de biologie moléculaire d'identifier la ou les mutations en clonant le ou les gènes mutés, et d'introduire cette mutation dans des souches industrielles.

Une approche directe peut également être effectuée par clonage du gène muté responsable du phénotype de sensibilité au froid dans le mutant présentant ce phénotype. Les résultats obtenus permettent de penser que la mutation responsable du phénotype de sensibilité au froid des mutants polyploides S47-12b1, L30-13 et L30-91 est à chaque fois dominante et monogénique.

On construit une banque d'ADN génomique d'un mutant d'une souche polyploide industrielle, pour laquelle, de préférence, le caractère dominant et monogénique de la mutation a été confirmé selon les techniques classiques de la biologie moléculaire dans un vecteur centromérique comme le vecteur YCp50, usuellement disponible, qui possède en plus le marqueur URA3. On transforme ensuite une souche de levure de laboratoire $ura3^-$, non sensible au froid, avec cette banque d'ADN et on recherche, parmi les transformants $URA3^+$, ceux qui ont acquis le caractère de sensibilité au froid par le procédé de sélection qui a conduit à la sélection des mutants sensibles au froid décrit ci-dessus, à savoir le procédé consistant à tuer les cellules ayant un métabolisme actif dans des conditions anaérobies strictes à une température comprise entre 10 et 15°C. Il est ensuite aisé de réisoler le plasmide d'un transformant exprimant le phénotype de sensibilité au froid, d'analyser le fragment d'ADN de levure cloné dans ce plasmide (cartographie de restriction, séquençage) pour identifier le gène et de réintroduire ce gène muté dans une souche de levure industrielle par les techniques d'intégration par recombinaison homologue dans un gène non essentiel à la croissance et à la fermentation ou dans le gène non muté.

D'autres techniques de biologie moléculaire peuvent être employées pour construire des souches industrielles transformées pour présenter le phénotype de sensibilité au froid des mutants objet de l'invention, et plus spécialement du mutant S47-12b1 c'est-à-dire pour obtenir des souches de levure rapide exprimant un phénotype de sensibilité au froid.

Une technique intéressante consiste à faire sporuler des souches industrielles, et notamment des souches de levure rapide, et appliquer le traitement de mutation et de sélection ci-dessus décrit aux ségrégeants ou haploïdes ainsi obtenus. Si après croisement des ségrégeants sélectionnés pour porter la mutation recherchée, il est vérifié que la mutation est dominante et monogénique, on procède comme décrit ci-dessus pour les polyploides portant une mutation monogénique et dominante. Si celle-ci est au contraire récessive et monogénique, on procède alors comme indiqué ci-après.

On isole, dans un premier temps, un clone $ura3^-$ du ségrégeant ou haploide portant une mutation de sensibilité au froid monogénique et récessive en l'étalant sur un milieu contenant du 5-fluoro-orotate ou en disruptant ses allèles URA3 par un marqueur positif ou encore en combinant ces deux techniques. Le mutant $ura3^-$ est ensuite transformé par une banque génomique d'une souche sauvage (wt) comme par exemple la banque d'ADN génomique construite dans le vecteur centromérique YCp50 et vendue par l'ATCC sous la référence 37415. On recherche ensuite, parmi les transformants $URA3^+$ obtenus, ceux qui ont perdu le phénotype de sensibilité au froid, en mettant à profit leur métabolisme beaucoup plus rapide en conditions anaérobies à 10°C que les ségrégeants mutés de départ.

L'identification du gène sauvage complémentant le phénotype de sensibilité au froid est réalisée par cartographie et séquençage. Cette identification permet aisément d'isoler l'allèle muté dans le ségrégeant portant une mutation de sensibilité au froid par la technique d'amplification génique (PCR) ou par la technique de "gap-filling". L'allèle muté sera, par la suite, utilisé pour substituer toutes les copies sauvages de ce gène, par recombinaison homologue, dans la souche industrielle que l'on souhaite rendre sensible au froid.

Cette variante est la méthode préférentielle selon l'invention pour obtenir des mutants intéressants par mutagenèse dirigée consistant à introduire dans des souches industrielles de levures de panification la mutation conférant le phénotype de sensibilité au froid. Elle a permis dans le contexte d'un ségrégeant de souche de levure rapide industrielle, bien adaptée à la fermentation du maltose, ce ségrégeant ayant lui-même cette propriété, de mettre en évidence qu'une mutation sur le gène YLR087c, selon le code adopté dans la banque résultant du séquençage systématique du génome de Saccharomyces cerevisiae, telle que synthétisée et coordonnée par le MIPS (Martinsried Institute for Protein Sequences - Max Planck Institute for Biochemistry - 82152 Martinsried - FRG) permettait, si tous les gènes YLR087c portent bien la mutation, d'obtenir des souches industrielles ayant le phénotype de sensibilité au froid en fermentation, quel que soit leur contexte génétique.

Ce résultat est particulièrement intéressant pour la construction de souches industrielles de levures de panification, et de manière générale de souches industrielles conduisant à des aliments ou boissons fermentées pour l'homme, et notamment du vin ou de la bière. Ce résultat est indépendant du contexte génétique, car la mutation de ce gène YLR087c a été identifiée sur une souche bien adaptée à la fermentation du maltose et réintroduite avec obtention du phénotype dans deux souches indépendantes l'une de l'autre et de la souche de départ. Cette mutation correspond à un phénotype de sensibilité au froid en fermentation, avec disparition totale du phénotype à plus de 20°C. Il est essentiel que la mutation ait été identifiée sur une souche industrielle ayant un phénotype de sensibilité au froid en fermentation. En effet, les trois mutations suivantes, décrites dans la littérature comme donnant des mutants "cold sensitive" ont été étudiées:

- le mutant prp28-1 décrit par STRAUSS, E.J. et GUTHRIE, C. (1991) "Genes and Development" 5, 629-641,
- la disruption d'une partie du gène LTE1 (Low Temperature Essential) décrit par WICKNER, R.B. et al. (1987) Yeast 3, 51-57,

- la mutation dirigée du gène BCY1 décrite par YAMANO, S. et al. (1987) "Mol.Gen.Genet." 210, 413-18.

Les mutants prp28-1 et lte1 ont été obtenus auprès des auteurs des publications citées ci-dessus. La mutation dirigée du gène bcy1 conduisant à un phénotype de sensibilité au froid a été reproduite dans une souche de laboratoire. Bien que ces trois mutations conduisent à un phénotype de non croissance à basse température (10°C) sur boîte de milieu gélosé comme décrit par les auteurs de ces travaux, aucun de ces trois mutants ne présentait un phénotype de sensibilité au froid en conditions de fermentation dans les tests fermentomètres de type A décrits. Ces trois mutations n'ont donc aucun intérêt pour obtenir des levures de panification, de vinification ou de brasserie.

L'invention a donc notamment pour objet:

- le ségrégeant HL13.2.30 déposé au CNCM sous le n° I-1841 qui donne des levures bien adaptées au maltose et présentant un phénotype de sensibilité en fermentation au froid,
- le gène muté tel qu'existant dans le plasmide YCp50-10.39mut dans la souche E.coli DH5α déposée au CNCM sous le n° I-1842,
- toute souche de levure industrielle de panification, dont tous les gènes YLR087c portent une mutation codon stop non létale conférant le phénotype de fermentation au froid.

Il est possible enfin d'obtenir des souches industrielles, selon la revendication 1, notamment des souches rapides et très rapides, possédant une sensibilité au froid telle que définie dans le cadre de la présente invention, en faisant exprimer dans cette souche de manière conditionnelle, c'est-à-dire uniquement à basse température (0 à 15°C voire 20°C), un gène dont l'expression a pour résultat une très forte diminution de l'activité fermentative de la souche concernée.

Des gènes ou allèles mutés de gènes dont l'expression conduit à une très forte diminution de l'assimilation des sucres par la levure et/ou une importante baisse de son activité fermentative, ont été décrits dans la littérature. L'expression de l'un de ces gènes (ou allèles mutés) sous la dépendance d'un promoteur conditionné par la température conduisant à une forte expression du gène à basse température et à une expression quasi-nulle du gène au-dessus de 20°C, comme par exemple les promoteurs de gènes codant pour des protéines "cold shock" dont plusieurs ont été décrits, est un moyen de construire directement une souche de levure sensible au froid, à partir des souches industrielles les plus performantes.

Des promoteurs conditionnels sont par exemple les promoteurs du gène TIP1 ou de gènes homologues de TIP1 ou leurs allèles mutés, décrits notamment dans l'article "Identification of cis-and trans-acting elements involved in the expression of cold shock inducible TIP1 gene of Yeast Saccharomyces cerevisiae", de MUNOS-DORADO J. et al., publié dans "Nucleic Acid Research", 1994, Vol. 22, No. 4, 560-568, ou encore dans l'article "Cold shock induction of a family of TIP1 related proteins associated with the membrane in Saccharomyces cerevisiae", de KOWALSKI, L. et al., paru dans "Molecular Microbiology" 15, No. 2 (January 1995), 341-353. Ces promoteurs conditionnels en fonction de la température, comme ceux de la famille du promoteur du gène TIP1 peuvent aussi être couplés à un gène codant pour un facteur de régulation transcriptionnelle ayant une action de type répresseur par exemple comme le gène MIG1. Ce gène MIG1 code pour une protéine en doigts de zinc qui intervient dans la voie principale de répression glucose et bloque la transcription des gènes qui ont dans leur promoteur une séquence consensus URS (Upstream Regulatory Sequence), comme décrit dans l'article publié par NEHLIN, J.O. et RONNE H. paru dans EMBO J. 1990, 9, 2891-2898 et ayant pour titre "Yeast MIG1 repression is related to the mammalian early growth response and Wilm's tumour finger proteins". Pour compléter la construction, une séquence consensus, reconnue par la protéine codée par le gène répresseur mis sous la dépendance d'un promoteur conditionnel, est insérée dans le promoteur d'un gène essentiel de la fermentation. Par exemple, la séquence consensus reconnue par la protéine codée par le gène MIG1 décrite dans l'article de NEHLIN et al. cité ci-dessus, est intégrée dans les promoteurs de tous les allèles du gène de la pyruvate kinase.

On peut également rechercher une expression conditionnelle d'un gène essentiel à la fermentation des levures, comme par exemple un gène de la glycolyse comme les gènes codant pour la pyruvate kinase ou encore la pyruvate decarboxylase. Cette expression conditionnelle peut être provoquée en clonant ce gène sous la dépendance d'un promoteur dont l'expression est quasi nulle en-dessous de 15°C et est normale à 30°C. Une méthode alternative intéressante pour obtenir une expression conditionnelle d'un gène est celle décrite dans l'article "Control of Gene Expression by Artificial Introns in Saccharomyces cerevisiae", de YOSHIMATSU et T., et VAGANA F., paru dans "Science" 1988, 244 (4910), 1346-1348.

Ceci étant, l'invention est illustrée par les exemples suivants:

## EXEMPLE 1

### Obtention et sélection de mutants

On sélectionne des souches de levure de panification du commerce, notamment les souches de levures pressées commercialisées en Europe, et plus particulièrement les souches utilisées pour la fabrication de levures pressées rapides destinées à des panifications de type français, sans sucre ajouté ou avec peu de sucre ajouté.
On les soumet au traitement de mutation suivant:
Les cellules récoltées en phase stationnaire sont mutagénisées par l'éthylméthylsulfonate (EMS) de façon à obtenir 10 à 20% de survie. Les cellules mutagénisées sont ensuite régénérées par une culture en milieu riche : YPG pendant 3 h à 30°C puis mises à incuber en conditions de fermentation anaérobie stricte pendant 48 h à 10°C (YPG). On ajoute alors de la nystatine ou tout agent inhibiteur des cellules en division et on incube de nouveau 2 h à 10°C.
Le traitement post-mutagenèse d'enrichissement peut avantageusement être répété 2 à 3 fois. La concentration en nystatine est ajustée pour obtenir un taux de survie de 0,01%. On étale la suspension cellulaire résultant de l'enrichissement à la nystatine à une dilution appropriée pour obtenir des colonies isolées des mutants obtenus.
On sélectionne les mutants ainsi obtenus en mesurant par rapport à la souche de départ non mutée, les sucres consommés.
Les mutants sont cultivés sur milieu YEG-AGAR pendant 24 heures. Les levures sont récoltées et les cellules de levure sont mises en suspension dans l'eau distillée, de manière à ce que cette suspension ait une densité optique de 15.
10 microlitres de cette suspension de levure sont incubés en présence de 100 microlitres de milieu de culture YPM et YPG/M sur plaque de microtitration.
Les plaques de microtitration sont incubées soit à 30°C pendant 8 heures, soit à 10°C pendant 60 heures.
A la fin du temps d'incubation, la fermentation du ou des sucres est bloquée par ajout du réactif au dinitrosalicylate de sodium qui sert à doser les sucres réducteurs par réaction colorimétrique après chauffage de la plaque de microtitration à 55°C pendant 30 minutes.
On sélectionne les mutants qui d'une part après 60 heures à 10°C ont fermenté au plus environ 60% du mélange glucose/maltose du milieu YPG/M et au plus 30% du maltose du milieu YPM par rapport aux sucres fermentés par la souche de départ non mutée et qui d'autre part à 30°C en 8 heures ont consommé au moins 90% des sucres consommés par la souche de départ non mutée.
Cette mesure de la consommation de sucre ou de sucres à basse température permet de quantifier le caractère de sensibilité au froid des souches et d'éliminer les mutants faiblement sensibles au froid. Une première élimination peut être faite à l'oeil. Les souches dont la fermentation est ralentie à 10°C donnent une coloration marron foncé, les souches mutées peu sensibles donnent une coloration orange indiquant un faible taux de sucres réducteurs résiduels. Cette première sélection à l'oeil permet un premier tri, elle est ensuite complétée par des mesures de consommation de sucre.
La mesure de la consommation de sucre ou de sucres à 30°C permet d'éliminer les mutants dont la fermentation est globalement affectée quelle que soit la température.
Ensuite, on vérifie par des tests de cultures les performances des souches ainsi sélectionnées. On peut faire des tests serai-anaérobies de laboratoire donnant une récolte suffisante pour permettre des mesures d'activité fermentative selon la série des tests A ci-dessus décrits. On peut également faire des cultures aérobies en volume réduit, puis de plus en plus important.
On vérifie que les mutants sont stables et peuvent être multipliés dans des conditions équivalentes et avec des rendements équivalents à la souche non mutée de départ, qui est une souche utilisée industriellement pour la production de levures de panification.
Dans le cadre de cet exemple, notamment trois souches ont été sélectionnées :

- la souche S47-12b1, mutant stable d'une souche de levure rapide utilisée communément en Europe pour la production de levures fraîches pressées, ce mutant stable S47-12b1 a été déposé au CNCM sous le n° I-1645 le 05-12-1995;
- les souches L30-13 et L30-91, mutants stables d'une souche de levure utilisée communément en Europe pour la production de levures fraîches pressées pour pâtes sucrées. Ces mutants stables ont été déposés au CNCM, le mutant L30-13 sous le n° I-1647, le mutant L30-91 sous le n° I-1646, ces deux dépôts ont été effectués le 05-12-1995.

Ces trois souches ont donné les résultats suivants dans les tests de sélection de consommation de sucre 60 h à 10°C sur plaque de microtitration, la lecture de densité optique étant faite avec un lecteur de plaque de marque Metertech Σ960®, Micro Elisa Autoreader dans les conditions décrites ci-dessus:

| 60 heures à 10°C | | | | | |
|---|---|---|---|---|---|
| Milieu | Souche commerciale levure rapide S47 | Mutant S47-12b1 | Souche commerciale levure pour pâte sucrées L30 | Mutant L30-13 | Mutant L30-91 |
| YP maltose | | | | | |
| DO: 2,25<br>% sucre consommé | 0,102<br>95,5 % | 2,02<br>9,8 % | 0,13<br>94 % | 1,71<br>24 % | 2,00<br>11% |
| YP maltose/ glucose | | | | | |
| DO: 2,63<br>% sucre consommé | 0,135<br>95 % | 1,19<br>55 % | 0,21<br>92 % | 1,13<br>57 % | 1,70<br>35 % |

| 8 heures à 30°C | | | | | |
|---|---|---|---|---|---|
| Milieu | Souche commerciale levure rapide S47 | Mutant S47-12b1 | Souche commerciale levure pour pâte sucrées L30 | Mutant L30-13 | Mutant L30-91 |
| YP maltose | | | | | |
| DO : 2,25<br>% sucre consommé | 0.15<br>93 % | 0.18<br>92 % | 0.183<br>92 % | 0,22<br>91 % | 0,25<br>89% |
| YP mattose/ glucose | | | | | |
| DO : 2,63<br>% sucre consommé | 0,12<br>95 % | 0,155<br>94 % | 0,17<br>93 % | 0.205<br>92 % | 0,23<br>91 % |

## EXEMPLE 2

**Culture des traie mutants sélectionnés selon l'exemple 1**

Les trois mutants stables S47-12b1, L30-13 et L30-91 ont été cultivés de la manière suivante.

A l'exception de l'emploi de procédés et matériels spécifiés ci-après, les souches ont été propagées en plusieurs stades de multiplication aérobie, la levure fraîche a été récoltée, lavée, filtrée à l'aide de matériels classiques employés en levurerie et selon les procédés de fabrication habituels comme les matériels et les procédés décrits dans l'ouvrage "Yeast Technology" de Gerald Reed and Henry J. Peppler (1973), The Avi Publishing Company Inc. ou dans le chapitre "Production of Baker's Yeast", Gerald Reed, publié par Prescott and Dunn's Industrial Microbiology, 4ème Edition, édité par Gerald Reed, The Avi Publishing Co. Inc., second printing 1983.

Il est particulièrement veillé à ce que tous les nutriments nécessaires en petites quantités à la levure, minéraux (macroéléments et oligoéléments), vitamines (biotine, vitamines de groupe B) soient présents au moins dans les quantités les plus grandes recommandées dans les ouvrages de référence cités ci-dessus. De manière générale, ces essais sont menés comme indiqué dans les précédents brevets de la Demanderesse, comme la demande de brevet européen n° 92401168.7 du 23-04-92. Il est notamment veillé à obtenir des levures bien lavées et à refroidir rapidement la crème et les levures filtrées à 2°C.

Le dernier stade de multiplication de la levure conduisant à une levure fraîche comprimée très active est mené plus spécifiquement de la manière suivante:

- dilution du milieu de culture en fin de multiplication commerciale:

$$\frac{\text{Poids du moût levuré dans la cuve}}{\text{Quantité de mélasse à 50 \% de sucres totaux exprimés en saccharose}} = 5{,}2$$

De préférence, ces essais sont menés avec un mélange de 90 % de mélasses de betterave, 10% de mélasses de canne, ces deux mélasses devant être de bonne qualité, c'est-à-dire de bonne pureté et ne pas contenir d'inhibiteurs ou de substances toxiques pour les levures. Il doit en effet être particulièrement vérifié par des essais de cultures témoins que les mélasses ne contiennent pas d'additifs toxiques parfois ajoutés lors du travail d'extraction et de purification du sucre en sucrerie. Le sucre des mélasses de betterave est mesuré par la méthode Clerget (détermination du saccharose par double polarisation), le sucre des mélasses de canne est déterminé par mesure enzymatique du saccharose, glucose et fructose réellement présents et l'ensemble de ces sucres est calculé en équivalent saccharose;

- taux de multiplication horaire moyen sur le dernier cycle de multiplication de 14 heures: 1,12 à 1,14
- taux maximum de bourgeons de la levure récoltée: 3%
- taux azote/matières sèches levure récoltée: 7%
- taux $P_2O_5$/matières sèches levure récoltée: 2,5%.

Ces essais ont donné des levures fraîches de panification à environ 32% de matières sèches ayant les caractéristiques suivantes. Tous les résultats donnés dans les tableaux sont pour des levures fraîches ramenées à 32% de matières sèches.

| Mesures à 30°C | | | | | |
|---|---|---|---|---|---|
| Souche | Rendement de culture sur équivalent mélasse à 50% de saccharose | Test $A_1$ 2h 30° C | Test $A_3$ 2h 30°C | Test $A_5$ 2h 30C | Test $A_6$ 2h 30°C |
| | | | | en ml de $CO_2$ | |
| souche S47 | 90 % | 140 | 120 | 90 | |
| souche S47-12b1 | 88 % | 115 | 97 | 65 | |
| souche L30 | 82 % | 88 | 125 | 110 | 165 |
| souche L30-13 | 87 % | 88 | 120 | 103 | 145 |
| souche L30-91 | 82 % | 70 | 117 | 98 | 135 |

| Mesures à 8°C avec remontée de température | | | | | |
|---|---|---|---|---|---|
| Souche | Test | 48 h 8°C | remontée à 30°C pendant 1 h 30 | 30° 1 h | Total 30° C 2 h |
| S47 | $A_1$ | 167 | 34 | 12 | 18 |
| S47-12b1 | $A_1$ | 44 | 48 | 54 | 118 |
| S47 | $A_5$ | 171 | 81 | 69 | 135 |
| S47-12b1 | $A_5$ | 13 | 42 | 35 | 76 |
| L30 | $A_1$ | 121 | 36 | 18 | 30 |
| L30-13 | $A_1$ | 12 | 50 | 47 | 100 |
| L30-91 | $A_1$ | 2 | 42 | 24 | 43 |
| L30 | $A_5$ | 174 | 68 | 66 | 127 |
| L30-13 | $A_5$ | 6 | 36 | 44 | 99 |
| L30-91 | $A_5$ | 1 | 23 | 47 | 98 |

Les tableaux ci-dessus permettent de calculer le rapport:

$$\frac{\text{dégagement de } CO_2 \text{ après 48 heures de fermentation à 8°C}}{\text{dégagement de } CO_2 \text{ après 2 heures de fermentation à 30°C}} \text{ en \%}$$

On obtient le tableau ci-dessous:

| Souche | Rapport dans le test $A_1$ | Rapport dans le test $A_5$ |
|---|---|---|
| S47 | 119 % | 190 % |
| S47-12b1 | 44÷115 = 38 % | ≈ 20 % |
| L30 | 138 % | > 158 % |
| L30-13 | 14 % | 6 % |
| L30-91 | 3 % | 1 % |

Les levures fraîches de panification obtenues dans l'ensemble de cet exemple sont stables dans un test de conservation 7 jours à 21°C.

**EXEMPLE 3**

**Culture du mutant stable S47-12b1 à environ 8% d'azote sur matières sèches**

La souche stable S47-12b1 et la souche témoin S47 sont cultivées dans les mêmes conditions que dans l'exemple 2, si ce n'est que:

- taux de multiplication horaire moyen sur le dernier cycle de multiplication de 14 heures: 1,18
- taux maximum de bourgeons de la levure récoltée: 5%
- taux azote/matières sèches levure récoltée: 8,2%
- taux $P_2O_5$/matières sèches levure récoltée: 2,7%.

On obtient les résultats suivants:

| Mesures à 30°C | | | | |
|---|---|---|---|---|
| Souche | Rendement de culture sur équivalent mélasse à 50% de saccharose | Test $A_1$ 2h 30°C | Test $A_3$ 2h 30°C | Test $A_5$ 2h 30C |
| | | | en ml de $CO_2$ | |
| souche S47 | 91 % | 170 | 155 | 105 |
| souche S47-12b1 | 91 % | 172 | 149 | 103 |

| Mesures à 8°C avec remontée de température | | | | | | |
|---|---|---|---|---|---|---|
| Souche | Test | 48 h 8°C | remontée à 30°C pendant | 30°C | | |
| | | | | 1 h | 2 h | 3 h |
| | | | | en ml de $CO_2$ | | |
| $S_{47}$ | $A_1$ | 195 | 32 | 10 | 8 | 2 |
| S47 | $A_5$ | 190 | 65 | 65 | 70 | 50 |
| S47-12b1 | $A_1$ | 66 | 50 | 62 | 53 | 11 |
| S47-12b1 | $A_5$ | 8 | 36 | 40 | 42 | 48 |

Cela permet de calculer les rapports de dégagement de $CO_2$ dans les tests $A_1$ et $A_5$:

$$\frac{\text{dégagement de } CO_2 \text{ après 48 heures de fermentation à } 8°C}{\text{dégagement de } CO_2 \text{ après 2 heures de fermentation à } 30°C}$$

| | | |
|---|---|---|
| S47 | test $A_1$ | 115 % |
| | test $A_5$ | 110 % |
| S47-12b1 | test $A_1$ | 66÷172 = 38,4 % |
| | test $A_5$ | 8÷103 = 8 % |

Les levures fraîches de panification obtenues avec ces deux souches sont stables de la même manière dans un test de conservation 7 jours à 21°C.

**EXEMPLE 4**

**Isolement d'un gène muté responsable du phénotype de sensibilité au froid dans le contexte d'une souche rapide adaptée au maltose, et clonage de ce gène dans des souches de levure**

On fait sporuler, selon les techniques habituelles, des souches industrielles de levure rapide, adaptées au maltose comme par exemple la souche NCYC 995, objet du brevet des Etats-Unis d'Amérique n° 4 396 632 du 2 août 1983 aux noms de Philippe Clément et Annie Loiez. On obtient ainsi un certain nombre d'haploides dont l'haploïde ou ségrégeant désigné sous le n° HL13.2. On soumet les haploïdes ainsi obtenus au traitement de mutation et de sélection de mutants décrits dans l'exemple 1. On sélectionne ainsi la souche haploïde dénommée HL13.2.30 sensible au froid. Ce mutant haploïde industriel HL13.2.30 est caractérisé par le fait qu'après culture, il permet d'obtenir une levure ayant les caractéristiques suivantes:

test $A_1$ 2 heures à 30°C : 132 ml de $CO_2$

$$\frac{\text{dégagement de } CO_2 \text{ dans le test } A_1 \text{ après 48 heures à } 8°C}{\text{dégagement de } CO_2 \text{ dans le test } A_1 \text{ après 2 heures à } 30°C} = 0{,}35$$

Ce mutant haploïde présente selon ces résultats une bonne adaptation au maltose du fait de son activité supérieure à 100 dans le test $A_1$ et un caractère de sensibilité au froid du fait du rapport calculé ci-dessus de 0,35. L'étude par croisement de la sensibilité au froid de ce mutant HL13.2.30 démontre qu'il s'agit d'une mutation car elle est transmissible et récessive. Le phénotype conféré par cette mutation ne dépend pas a priori du contexte génétique de la souche.

Compte tenu de ces résultats, le clonage du gène responsable du phénotype de sensibilité au froid de cette souche haploïde mutée désignée sous le n° HL13.2.30 a été entrepris selon la stratégie générale suivante dont chaque étape sera reprise de manière plus détaillée ensuite. La souche haploïde mutée HL13.2.30 a été déposée au Centre National de Culture de Microorganismes (CNCM), Institut Pasteur Paris, sous le n° I-1841 le 30-01-1997.

1) Stratégie générale

- Isolement d'un mutant $ura3^-$ de la souche HL13.2.30 afin de pouvoir la transformer avec la banque d'ADN génomique référence ATCC 37415 construite dans le vecteur centromérique YCp50 (marqueur URA3). [ROSE, M.D. et al. (1987) Gene 60, 237-243].
- Transformation d'un mutant HL13.2.30 $ura3^-$ par la banque d'ADN génomique et isolement de clones devenus $URA3^+$ (clones ayant reçu un plasmide).
- Recherche de clones transformants ayant perdu le phénotype de sensibilité au froid.
- Vérification des clones ayant perdu le phénotype de sensibilité au froid.
- Isolement et caractérisation du plasmide après réisolement de ce dernier à partir d'un transformant ayant perdu le caractère de sensibilité au froid.

- Retransformation de la souche HL13.2.30 ura3⁻ avec le plasmide réisolé et vérification qu'il complémente effectivement la mutation.
- Localisation chromosomique du fragment d'ADN génomique inséré dans le plasmide par séquençage des extrémités 3' et 5' de l'insert.
- Identification de l'ORF (phase de lecture ouverte = Open Reading Frame) responsable de la levée du caractère de sensibilité au froid.
- Isolement du gène portant la mutation à partir du mutant HL13.2.30.
- Séquençage du gène muté et de son allèle "wt" (= wild type ou sauvage) de la souche HL13.2 pour identifier et localiser la mutation.
- Retransformation de la souche HL13.2.30 ura3⁻ (mutant) avec le gène muté de manière à vérifier qu'il ne s'agit pas d'un gène suppresseur.
- Réintroduction (par recombinaison homologue) du gène muté dans un ségrégeant non sensible au froid (souche de laboratoire n=16 chromosomes), vérification de l'obtention du phénotype de sensibilité au froid et ainsi obtention de la démonstration de la monogénicité et de la non dépendance du contexte génétique de la souche mutée d'origine.

Toutes les constructions ont été réalisées selon les techniques habituelles et notamment selon l'ouvrage "MOLECULAR CLONING", J. Sambrook, E.F. Fritsch, T. Maniatis, Cold Spring Harbor Laboratory Press 1989.

2) Isolement d'un mutant ura3⁻ de la souche HL13.2.30

Cette étape a été nécessaire afin de pouvoir transformer la souche HL13.2.30 par la banque génomique et sélectionner les transformants qui sont devenus $URA^+$ par l'introduction du plasmide.
La démarche entreprise pour rendre ura3⁻ la souche HL13.2.30 est une disruption des allèles URA3 de cette souche par le gène de résistance à la phléomycine. A été construite une cassette de disruption du gène URA3 par la phléomycine dans le plasmide YIp lac 211 (URA3) [GIETZ, R.D. and SUGINO, A. (1988) Gene 74, 527-534].
La construction (schématisée à la figure 1) comprend les étapes suivantes:

- préparation du fragment pTEF1 / Tn5 Ble / t CYC1 (gène de résistance à la phléomycine) par une double hydrolyse Hind III / Kpn I du plasmide pUT332 [GATIGNOL, A. et al. (1987) "Mol. Gen. Genet." 207, 342-348]. Le fragment est récupéré sur gel d'agarose LMP (Low Melting Point), purifié par le kit QIAEX (vendu par QIAGEN) et rendu bouts francs par la T4 DNA Polymerase.
- ouverture du plasmide YIp lac 211 par l'enzyme Stu I qui coupe à l'intérieur du gène URA3 (site unique) et génère des extrémités franches. Le plasmide ouvert est ensuite déphosphorylé par la CIF (Calf Intestine Phosphatase) pour empêcher la recircularisation du plasmide lors du clonage.
- clonage du fragment pTEF1 / Tn5 Ble / t CYC1 bouts francs dans le plasmide YIp lac 211 - Stu I - déphosphorylé par l'action de la T4 DNA ligase.
- transformation de la souche E.coli DH5$\alpha$ (vendue par LIFE TECHNOLOGIES) par le ligat précédent et isolement de clones ayant intégré le gène de résistance à la phléomycine.
- libération de la cassette de disruption ura3::$PH^R$ (gène URA3 disrupté par le gène de résistance à la phléomycine) par une double hydrolyse Nde I / Nsi I, obtention de la libération d'un fragment de 2,1 kb = ura3::$PH^R$.

La souche HL13.2.30 a été transformée par électroporation avec le fragment ura3::$PH^R$ de 2,1 kb (1 $\mu$g). Plusieurs clones $PH^R$ ont été obtenus mais tous étaient encore $URA^+$ démontrant ainsi l'existence d'un deuxième allèle URA3 (au moins) dans la souche HL13.2.30.
Une deuxième disruption a été effectuée avec la même cassette d'ADN mais la sélection a cette fois été réalisée sur un milieu minimum + uracile + 5FOA (5-Fluoro Orotate), milieu permettant une sélection directe des clones ura3⁻ : plusieurs clones $FOA^+$ ont été obtenus, ils étaient tous ura3⁻. La souche HL13.2.30 contient donc deux copies du gène URA3.

3) Transformation de la souche HL13.2.30 ura3⁻ par la banque génomique

Plusieurs transformations de la souche HL13.2.30 ura3⁻ ont été réalisées avec l'ADN plasmidique de la banque génomique ATCC 37415 construite dans le vecteur centromérique YCp50. Les transformations ont été réalisées par la technique à l'acétate de lithium : $10^8$ cellules / transformation en présence de 630 ng d'ADN (telle que décrite par GIETZ, D. et al. (1992) "Nucl. Acids Res." 20, 1425).
Plus de 5000 clones transformants ont été obtenus dont 4500 ont été criblés dans un test de présélection constitué par un test gouttes sur milieu glucose 0,1% de préférence avec antimycine (5 $\mu$g/ml).
Les transformants sont cultivés sur milieu minimum YNB-agar DIFCO® contenant 2% de glucose pendant 24 heures. Les levures récoltées sont mises en suspension dans l'eau distillée; la suspension est ajustée à densité optique 1. Des gouttes de 5 microlitres de cette suspension sont déposées sur milieu minimum YNB-agar DIFCO® renfermant 0,1% de glucose et 5 μg/ml d'antimycine A (référence A 8674 dans le catalogue SIGMA), l'antimycine A étant un agent bloquant la respiration. Les boîtes contenant ce milieu minimum et ainsi ensemencées sont incubées à 10°C pendant 10 jours. On sélectionne les transformants qui se sont multipliés grâce à l'énergie produite par fermentation, puisque leur respiration est bloquée par l'antimycine A, plus rapidement que le mutant de départ HL13.2.30 $ura3^-$ transformé par le plasmide YCp50 vide, c'est-à-dire sans insert.

Cette présélection a permis de retenir une cinquantaine de clones présentant une croissance améliorée à 10°C par rapport au témoin, c'est-à-dire présentant une réversion vers le phénotype sauvage. Tous ces clones ont fait l'objet d'une étude plus poussée afin de voir si, parmi ces candidats, un clone avait effectivement perdu son caractère de sensibilité au froid par transformation avec la banque.

Les tests complémentaires qui ont été effectués sur ces clones sont les suivants:

- Test glucose à 10°C : mesure de la consommation du glucose à 10°C en conditions de fermentation comparée aux témoins HL13.2.30 $ura3^-$ / YCp 50 (mutant sensible au froid) et HL13.2 (souche sauvage) de manière à vérifier l'existence ou non du phénotype de sensibilité au froid en fermentation, ce test ayant été corrélé avec des tests au fermentomètre de Burrows et Harrison de type A tels que décrits ci-dessus.

La description du test glucose est la suivante : les différents clones sont cultivés sur milieu minimum pendant 24 h. Les levures sont récoltées et les cellules de levures sont mises en suspension dans l'eau distillée, de manière à ce que cette suspension ait une densité optique de 15. 10 microlitres de cette suspension de levures sont incubés en présence de 100 microlitres de milieu minimum (yeast nitrogen base, w/o Amino-acid DIFCO®) contenant 0,02% de glucose, sur plaque de microtitration. Les plaques de microtitration sont incubées soit à 30°C pendant 4h, soit à 10°C pendant 20h. A la fin du temps d'incubation, le glucose non fermenté par les levures est révélé par une réaction colorimétrique, à la glucose oxydase (Diagnostic Kits Sigma n° 315-100). Ce test est une variante du test sur plaque de microtitration avec révélation de la consommation de sucre au dinitrosalicylate de sodium décrit dans l'exemple 1.

- Fermentomètre 48h à 8°C selon test $A_1$ de manière à vérifier la perte du caractère de sensibilité au froid.
- Identification des clones par une technique de PCR fingerprinting (Ness et al., 1993, "J.Sci.Food Agric.", 62, 89-94) de manière à vérifier qu'il ne s'agit pas de contaminants.
- Croissance sur milieu YEG-Agar + phléomycine 100 μg/ml de manière à vérifier la présence du caractère de résistance à la phléomycine apporté lors de la disruption des allèles URA3 de la souche HL13.2.30.
- Vérification de la présence d'un plasmide : isolement de l'ADN total et retransformation de la souche E.coli DH5α pour isoler le plasmide.

Parmi la cinquantaine de clones repérés par le test gouttes, un clone ayant satisfait à tous ces tests a été retenu: le clone YCp50-10.39. Ce clone a fait l'objet d'une caractérisation complète.

4) Etude du clone YCp50-10.39

a) Confirmation de la perte du phénotype de sensibilité au froid par la transformation

De manière à vérifier que la perte du caractère de sensibilité au froid était bien liée à la présence d'un fragment d'ADN génomique apporté par le plasmide, nous avons comparé, au fermentomètre à 8°C, les dégagements gazeux des souches suivantes:

| | |
|---|---|
| HL13.2 : | témoin |
| HL13.2.30 $ura3^-$[YCp50] : | témoin levure sensible au froid (souche transformée par le plasmide "vide"). |
| HL13.2.30 $ura3^-$[YCp50-10.39]: | clone YCp50-10.39 isolé après transformation de la souche HL13.2.30 $ura3^-$ (levure sensible au froid) par la banque d'ADN génomique. |
| HL13.2.30 $ura3^-$[YCp50-10.39 RT]: | clone isolé après retransformation de la souche HL13.2.30 $ura3^-$ par le plasmide isolé du clone YCp50-10.39 originel. |
| HL13.2.30 $ura3^-$[YCp50-10.39 FOA]: | clone isolé sur 5-FOA ($ura^-$) après perte du plasmide sur milieu riche du clone YCp50-10.39 originel. |

Des mesures de dégagements gazeux ($CO_2$) au fermentomètre de Burrows et Harrison dans le test $A_1$ à 8°C pendant 24 ou 48 heures montrent:

- que les trois souches HL13.2, H13.2.30 ura3⁻ [YCp50-10.39] et HL13.2.30 ura3⁻[YCp50-10.39RT] donnent un dégagement de $CO_2$ du même ordre de grandeur, et donc comme la souche HL13.2 ne présentent aucun phénotype de sensibilité au froid
- tandis que les deux souches HL13.2.30 ura3⁻[YCp50] et HL13.2.30 ura3⁻[YCp50-10.39FOA] donnent toutes les deux un dégagement de $CO_2$ environ 3 fois plus faible que celui du groupe formé par les 3 souches ci-dessus, ces deux souches présentent donc un phénotype de sensibilité au froid.

Ces résultats, vérifiés plusieurs fois et sur plusieurs clones indépendants, montrent sans ambiguïté que le caractère de sensibilité au froid de la souche HL13.2.30 ura3⁻ est complémenté dans le clone YCp50-10.39 et que la complémentation est bien liée à la présence du plasmide recombinant.

b) Isolement et caractérisation du plasmide présent dans le clone YCp50-10.39

Le plasmide du clone transformant YCp50-10.39 a été isolé après transformation de la souche E. coli DH5α avec l'ADN total du clone YCp50-10.39 préparé par kit QIAGEN.

Une cartographie sommaire du plasmide a permis de montrer que:

- le plasmide contenait bien un insert,
- la taille de l'insert était supérieure à 15 kb.

Les extrémités 5' et 3' de l'insert ont été séquencées afin de localiser ce fragment d'ADN génomique dans le génome de Saccharomyces cerevisiae. La séquence obtenue a permis de déterminer, en consultant sur INTERNET la banque de données du MIPS (Martinsried Institute for Protein Sequences - Max Planck Institute for Biochemistry - 82152 Martinsried - FRG) à l'adresse sur Internet:
http://speedy.mips.biochem.mpg.de/mips/yeast/index.htmlx que :

- l'insert correspondait à un fragment d'ADN du chromosome XII,
- la taille exacte de l'insert est 18178 pb,
- le fragment d'ADN s'aligne sur le chromosome XII (à droite de centromère) au niveau des nucléotides 305116 à 323293.

L'insert comprend les 5 phases ouvertes de lecture (ou ORF) complètes suivantes numérotées ci-après en chiffres romains de I à V:

| | | |
|---|---|---|
| I | YLR087c | - ORF de 8874 pb (paires de base) correspondant à une protéine de 2958 AA (acides aminés)<br>- protéine dite "hypothétique" dans la mesure où elle n'a jamais été isolée - aucune homologie ou similarité connue<br>- 4 zones transmembranaires |
| II | YLR088w | - ORF de 1842 pb : protéine de 614 AA<br>- gène connu : GAA1 (= END2) codant pour une protéine intervenant dans la translocation des ancres GPI (N - glucosyl phosphatidyl inositol) sur les protéines attachées à la membrane par ces ancres<br>- la disruption de ce gène est létale |
| III | YLR089c | - ORF de 1776 pb : protéine de 592AA<br>- protéine présentant une forte homologie avec les alanine transaminases |
| IV | YLR090w | - ORF de 1377 pb : protéine de 459 AA<br>- protéine présentant des homologies avec la protéine Dna J (= heat shock protein chez E.coli)<br>- expression non mise en évidence chez *S. cerevisiae* pourrait être un gène cryptique<br>- délétion du gène viable : aucun phénotype |
| V | YLR091w | - ORF de 879 pb : protéine de 293 AA<br>- protéine hypothétique. Aucune homologie connue. |

Cette notation YLR087c pour le premier ORF correspond au code suivant dans la banque de données:

Y = Yeast = levure Saccharomyces cerevisiae

L = Chromosome XII
R = à droite du centromère
087 = ORF n°087
c = protéine codée par le brin Crick

c) Identification du gène responsable de la levée du caractère de sensibilité au froid

De manière à préciser exactement le gène responsable de la complémentation du caractère de sensibilité au froid de la souche HL13.2.30 ura3⁻, nous avons réalisé divers sous-clonages et délétions pour arriver au résultat.

- les délétions ont été réalisées directement à partir du plasmide YCp50-10.39
- les sous-clonages des fragments de l'insert de 18178 pb ont été effectués dans le plasmide YCp lac33 [GIETZ, R.D. and SUGINO, A (1988) Gene 74, 527-534].

La stratégie employée est présentée à la figure 2 avec les résultats obtenus avec le test glucose ("+" : complémentation du caractère de sensibilité au froid; ''-" : non complémentation). Ces résultats ont été confirmés par les mesures de dégagement gazeux au fermentomètre après 24 ou 48 h à 8°C selon le test $A_1$.

| | |
|---|---|
| HL13.2 : | témoin non sensible au froid. |
| HL13.2.30 ura3⁻[YCp50-10.39] : | clone YCp50-10.39 isolé après transformation de la souche HL13.2.30 ura3⁻ (sensible au froid) par la banque d'ADN génomique. |
| HL13.2.30 ura3⁻ [YCp lac33-YLR087c] : | souche HL13.2.30 ura3⁻ transformée par le plasmide YCp lac33 dans lequel a été cloné le gène YLR087c sauvage (fragment NaeI - NaeI du plasmide de YCp50.10.39). |
| HL13.2.30 ura3⁻[YCp50-10.39mut] : | souche HL13.2.30 ura3⁻ transformée par le plasmide [YCp50-10.39mut] obtenu par la technique de "gap-filling" décrite au paragraphe d) ci-après. Ce plasmide contient le gène YLRO87c portant la mutation. |
| HL13.2.30 ura3⁻[YCp50] : | souche HL13.2.30 ura3⁻ transformée par le plasmide YCp50 "vide" (plasmide d'origine de la banque génomique ATCC). |
| HL13.2.30 ura3⁻[YCp lac33] : | souche HL13.2.30 ura3⁻ transformée par le plasmide YCp lac33 "vide" (plasmide centromérique URA3 ayant servi aux divers sous-clonages). |

Les dégagements au fermentomètre à 8°C selon le test $A_1$ de ces différentes souches ont donné les résultats suivants:

- les trois souches HL13.2, HL13.2.30 ura3⁻[YCp50-10.39], HL13.2.30 ura3⁻[YCp lac33-YLR087c] donnent un dégagement gazeux de $CO_2$ du même ordre de grandeur et donc comme la souche témoin HL13.2 ne présentent aucun phénotype de sensibilité au froid;
- tandis que les trois souches HL13.2.30 ura3⁻[YCp50-10.39mut], HL13.2.30 ura3⁻[YCp50]et HL13.2.30 ura3⁻[YCp lac33] donnent un dégagement gazeux de $CO_2$ environ 3 fois plus faible que le groupe précédent et présentent donc un phénotype de sensibilité au froid.

Des résultats du test glucose et du fermentomètre à 8°C, il ressort clairement que le gène responsable de la complémentation est le gène YLR087c, codant pour une protéine de 2958AA (Acides Aminés), de fonction totalement inconnue et de localisation vraisemblablement membranaire.

d) Isolement du gène YLR087c muté

De manière à vérifier qu'il s'agissait bien du gène recherché, responsable du phénotype et non d'un gène suppresseur, nous avons cloné le gène muté de la souche HL13.2.30 ura3⁻ par la technique du " gap-filling " [IWASAKI, T. et al. (1991) Gene 109, 81-87] ou " Allele rescue " [ORR-WEAVER, T.L et al. (1983) "Methods Enzymol." 101, 228-245]. Le principe de la méthode est donné plus particulièrement dans la figure 2 "Basic strategy for cloning GAP regions", page 83 de l'article de IWASAKI, T. et al.

Cette méthode a été appliquée à la souche HL13.2.30 ura3⁻ en la transformant avec le plasmide YCp50-10.39 Δ Sac I (cf. figure 2) digéré par Esp I et Afl II. L'utilisation d'un plasmide avec insert délété (par Sac I) est nécessaire pour vérifier qu'il y a bien eu remplissage (et qu'il ne s'agit pas d'une hydrolyse incomplète du plasmide). 3 clones correspondant au résultat attendu (nommés GF2, GF4 et GF5) ont été obtenus.

La retransformation de la souche HL13.2.30 ura3⁻ avec l'ADN isolé du clone GF2 (= YCp50-10.39 mut.) a permis de montrer que les transformants $URA^+$ obtenus étaient tous sensibles au froid.

Ce résultat démontre que le gène YLRO87 n'est pas un gène suppresseur du caractère de sensibilité au froid de la souche HL13.2.30 $ura3^-$. En effet, si YLRO37c était un gène suppresseur (donc non muté), son expression épisomique après transformation de la souche HL13.2.30 $ura3^-$ par l'ADN de GF2 aurait conduit à une levée du caractère de sensibilité au froid.

Le plasmide YCp50-10.39mut dans la souche Escherichia coli DH5α a été déposé à la Collection Nationale de Cultures de Microorganismes, Institut Pasteur, sous le n° I-1842 le 30 janvier 1997.

e) Détermination de la séquence nucléotidique du gène YLR087c muté - Identification de la mutation :

Le gène YLR087c de la souche HL13.2.30 (déposée au CNCM sous le numéro I-1841) a été entièrement séquencé par la technique de séquençage direct sur produits de PCR obtenus par amplification de fragments d'environ 1 kb couvrant tout le gène. Tous les fragments de PCR séquencés se chevauchent afin qu'il ne persiste aucune zone non séquencée du gène. La séquence a été déterminée à partir des extrémités des ORFS YLR086w et YLR088w contigus au gène YLR087c.

La séquence obtenue a été comparée avec la séquence du gène YLR087c publiée dans les bases de données et qui peut être obtenue auprès du MIPS (Martinsried Institute for Protein Sequences - Max Planck - Institute for Biochemistry - 82152 Martinsried - FRG) et notamment au site Internet :
http://speedy.mips.biochem.mpg.de/mips/yeast/index.htmlx.

La comparaison des séquences a permis d'identifier 35 changements (ou indéterminations) de nucléotides dans la région codante du gène :

- 27 changements (ou indéterminations) n'induisant aucune modification de l'acide aminé dans la chaîne polypeptidique
- 7 changements induisant le changement de l'acide aminé
- 1 changement provoquant l'apparition d'un codon STOP (position n°7865 du gène - séquence SEQ ID No. 1, cette position correspond à la position n°308242 dans le chromosome XII dans la séquence du MIPS).

De façon à vérifier s'il s'agissait ou non de mutations vraies, le gène YLR087c de la souche HL13.2 (souche sauvage à partir de laquelle le mutant HL13.2.30 a été obtenu) a été séquencé selon la même stratégie au niveau des régions où des modifications avaient été identifiées.

Toutes les modifications citées ci-dessus ont été retrouvées dans la séquence du gène sauvage sauf la modification identifiée à la position n°7865 (C → G] conduisant au changement Ser → STOP (TCA → TGA).

Cette modification, qui correspond à une mutation vraie du gène YLR087c de la souche HL13.2.30 (puisque non retrouvée dans la séquence du gène sauvage), provoque un arrêt de traduction de la protéine codée par ce gène occasionnant la synthèse d'une protéine tronquée de 2496AA chez le mutant alors que la protéine normale synthétisée par une souche sauvage est constituée de 2958AA(Acides Aminés).

L'ADN plasmidique des clones [YCp50-10.39] et [YCp50-10.39mut] a également été séquencé sur 120 pb (séquençage double brin sur l'ADN plasmidique) au niveau de la région de l'insert couvrant le codon STOP. Le résultat obtenu a permis de confirmer l'existence de la mutation identifiée sur l'ADN génomique. La séquence lue (position 7864 → 7866 dans la séquence SEQ ID No. 1) est:

- TCA (Ser) pour le clone [YCp50-10.39]
- TGA (STOP) pour le clone [YCp50-10.39mut]

Le clone DH5α[YCp50-10.39mut] renfermant le plasmide contenant le gène YLRO87c muté déposé au CNCM sous le n° I-1842, comporte donc effectivement cette mutation.

La séquence du gène YLR087c muté de la souche HL13.2.30 déposée au CNCM sous le numéro I-1841, obtenue par séquençage direct est donnée (SEQ ID No. 1), ainsi que celle de la protéine codée par ce dernier (SEQ ID No. 2).

5) Réintroduction d'un fragment d'ADN portant la mutation "STOP" dans une souche de levure et étude du phénotype conféré

a) Construction du plasmide $pUC_9$-3162ΔG418

De manière à vérifier que la seule mutation STOP identifiée par le séquençage était nécessaire et suffisante pour conférer un phénotype de sensibilité au froid à une levure, nous avons sous-cloné un fragment de 3162 pb (fragment HindIII, nucléotides 6035-9196 du gène YLR087c muté, dans la séquence SEQ ID No. 1, renfermant la mutation "STOP" en position 7865). Un marqueur de résistance à la généticine (G418) a ensuite été introduit au site Eco RV unique (position 8379 du gène) de façon à pouvoir sélectionner des levures qui ont été transformées par ce fragment par recombinaison homologue.

Le détail de la construction est donné ci-dessous (et est expliqué de façon schématique à la figure 3):

- Isolement du fragment Hind III de 3162 pb par hydrolyse du plasmide YCp50-10.39mut contenant le gène YLR087c muté. Le fragment est séparé par électrophorèse sur agarose à bas point de fusion (agarose LMP) et purifié par kit QIAEX QIAGEN).
- Clonage du fragment Hind III de 3162 pb décrit ci-dessus dans le plasmide $pUC_9$ au niveau du site unique Hind III présent dans le site multiple de clonage de ce vecteur. On obtient alors le plasmide nommé $pUC_9$-3162.
- Ouverture du plasmide $pUC_9$-3162 au niveau du site unique Eco RV inclus dans le fragment de 3162 pb (position n°8379 du gène - SEQ ID No. 1). Cette enzyme (Eco RV) libère des extrémités franches qui sont ensuite déphosphorylées par l'action de la CIP (Calf Intestine Phosphatase).
- Préparation du fragment d'ADN pPGK/Tn903/tPGK correspondant au gène de résistance à la généticine (G418) par double hydrolyse Hind III/AccI du plasmide $pUC_{19}$-G418. Le fragment de 3,1 kb est récupéré par électrophorèse sur gel d'agarose LMP suivie d'une purification par kit QIAEX. Ce fragment est ensuite rendu bouts francs par l'action de l'ADN Polymérase (fragment de Klenow) en présence des 4 dNTP.
- Le gène de résistance au G418 rendu bouts francs (description ci-dessus) est ensuite cloné au site Eco RV du plasmide $pUC_9$-3162 par l'action de la T4 DNA ligase. Après transformation de la souche DH5$\alpha$ d'E.coli, on obtient le plasmide $pUC_9$-3162$\Delta$G418 qui porte un fragment d'ADN d'environ 6,2 kb renfermant la mutation STOP et le gène de résistance au G418.

b) <u>Transformation des souches haploides de levures M5 et OL1</u>

- L'hydrolyse du plasmide $pUC_9$-3162$\Delta$G418 permet de libérer un fragment d'ADN linéaire de 6,2 kb qui peut, par recombinaison homologue, remplacer 1a partie correspondante d'un gène YLR087c sauvage par le même fragment portant la mutation STOP. L'intégration se fait par remplacement de gène, par un mécanisme de double "crossing-over" comme décrit par RODSTEIN, R. (1991) Methods in Enzymology, 194, 281-301. Le gène de résistance au G418, introduit 514 pb après la mutation STOP (dans le sens 5'$\rightarrow$3' de lecture de l'ORF) n'est utile que pour la sélection des transformants.
- Deux souches indépendantes haploïdes de laboratoire de Saccharomyces cerevisiae : M5 (MATA, ura3, trp1, leu2) et OL1 (MAT$\alpha$, leu2, his 3, ura3) ont été transformées avec l'ADN linéaire 3162$\Delta$G418 (fragment Hind III de 6,2 kb du plasmide $pUC_9$-3162$\Delta$G418) par électroporation selon la méthode décrite par MEILHOC, E et al. (1990) Biotechnology, 8, 223-227. Les conditions d'électroporation sont les suivantes:
  - 2500 V/cm
  - $10^8$ cellules dans 50 $\mu$l
  - 1 $\mu$g d'ADN linéaire.

Plusieurs centaines de clones transformants résistants au G418 ont été obtenus avec ces deux souches. L'analyse de 20 clones pris au hasard pour chacune de ces deux souches a permis de montrer que tous ces clones avaient un phénotype de sensibilité au froid.

La souche M5 est un haploïde vrai 16n (16 chromosomes) issu de la souche M5 2n décrite par SCHAAFF et al. (1989) Curr.Genet. 15, 75-81. La souche OL1 est un haploïde vrai 16n décrit par BOY-MARCOTTE, E. et JACQUET, M. (1982) Gene 20, 433-440.

Ce résultat montre que lorsque la mutation est réintroduite dans une souche de levure haploide par la technique de remplacement de gène, elle confère un phénotype de sensibilité au froid aux clones transformés.

6) <u>Construction de souches industrielles de levure de panification sensibles au froid par introduction de la mutation STOP dans tous leurs gènes YLR087c</u>

La mutation STOP identifiée dans le gène YLR087c et décrite ci-dessus est une mutation récessive, transmissible et non dépendante d'un contexte génétique particulier de la souche (cf transformation de deux souches de laboratoire indépendantes décrites ci-dessus en 5).

Pour construire une souche de levure industrielle sensible au froid, il est nécessaire d'introduire la mutation STOP identifiée dans tous les allèles du gène YLR087c présents dans la souche pour lui conférer le phénotype de sensibilité au froid. Les constructions décrites en 5 ci-dessus ($pUC_9$-3162 et $pUC_9$-3162ΔG418) permettent d'arriver à cet objectif. Sur le même modèle de la construction du vecteur $pUC_9$-3162ΔG418, on peut introduire d'autres marqueurs positifs comme par exemple les gènes de résistance à la phléomycine, à la cycloheximide, à des herbicides, à des métaux (Cu, Cd). Selon la même procédure (remplacement de gène) que celle décrite ci-dessus, tous les allèles sauvages du gène YLR087c peuvent être transformés de façon à introduire la mutation STOP et un marqueur positif différent pour chaque allèle du gène YLR087c. L'obtention d'une souche "propre" ne contenant que le seul changement du nucléotide 7865 de la séquence SEQ ID No. 1 (mutation STOP, position 308242 du chromosome XII de Saccharomyces cerevisiae selon la banque de données du MIPS) est ensuite réalisée en transformant plusieurs fois la souche par une technique de co-transformation mettant en jeu le fragment d'ADN linéaire de 3162 pb (obtenu par digestion HindIII du plasmide $pUC_9$-3162) et un plasmide de type YEP contenant un marqueur positif autre que ceux utilisés dans les transformations par intégration. Les clones obtenus après co-transformation sont ensuite répliqués sur boîtes contenant un milieu approprié renfermant chacune une substance toxique (antibiotique, herbicide,...) utilisée lors des étapes d'intégration. Sont sélectionnés les clones qui ont perdu au moins un des caractères de résistance. A l'issue de cette sélection par répliques, le plasmide réplicatif utilisé lors de la co-transformation est éliminé en cultivant le(s) clone(s) sélectionné(s) sur milieu riche sans aucune pression de sélection. On retient, à l'issue de cette culture, un clone ayant perdu le caractère de résistance porté par le plasmide.

La répétition de cette séquence d'événements de transformation permet de remplacer un à un les allèles mutés portant un marqueur de résistance par des allèles portant la mutation seule sans autre ADN hétérologue de type marqueur de résistance.

De préférence on utilisera une souche de levure de boulangerie possédant un nombre limité de copies du gène YLR087c. De préférence encore, on transformera dans un premier temps les parents (ségrégeants) de cette souche que l'on recroisera une fois que le phénotype de sensibilité au froid aura été obtenu pour chacun des parents.

De façon alternative, on peut mettre à profit la méthode de sélection par enrichissement à la nystatine décrite dans l'exemple 1.

Dans ce cas, on procède également par cotransformation en mettant en jeu l'ADN linéaire portant la mutation STOP (fragment Hind III de 3162 pb du plasmide $PUC_9$-3162) et un plasmide réplicatif de type YEp portant un marqueur positif (résistance au G418 ou à la phléomycine). Les clones résistants à l'antibictique obtenus après la co-transformation sont rassemblés et soumis au protocole d'enrichissement à la nystatine qui permet de sélectionner des cellules ayant le phénotype de sensibilité au froid. Le plasmide réplicatif portant un marqueur de résistance est ensuite éliminé par culture d'un clone (sélectionné comme sensible au froid) sur milieu riche sans pression de sélection (perte du plasmide). Cette alternative permet de sélectionner directement des événements d'intégration (par remplacement de gène) multiples qui auraient pu avoir lieu. Là encore, il est préférable de travailler avec une souche possédant un nombre de copies limité des gènes YLR087c ou mieux encore de transformer préalablement les parents de cette souche.

La vérification de la construction peut être contrôlée en séquençant un produit de PCR couvrant la zone siège de la manipulation génétique souhaitée.

Une troisième alternative peut également être employée pour arriver à l'objectif souhaité qui est de remplacer chaque allèle YLR087c d'une souche industrielle par des allèles portant la mutation STOP. Cette méthode est décrite dans l'article publié par ALANI, E. et al. (1987) "Genetics" 116, 541-545. Elle consiste à flanquer un marqueur positif comme par exemple le gène de résistance au G418 par deux séquences répétées directes de préférence des séquences identiques au gène YLR087c. La culture d'un transformant G418 résistant sur un milieu riche sans G418 permet une excision du marqueur par un mécanisme de "pop-out". La répétition de cette séquence d'intégration / excision permet, comme cela est décrit dans l'article cité ci-dessus, de remplacer un à un chacun des allèles sauvages par des allèles portant la mutation STOP.

Cet exemple est non limitatif, et on peut penser qu'un résultat identique peut être obtenu par des mutations non létales d'autres gènes comparables au gène YLR087c codant pour des protéines membranaires, étant entendu que chacun des gènes similaires ou ayant les mêmes propriétés que le gène YLR087c doit porter la même mutation non létale. Ces gènes similaires au gène YLR087c sont par exemple :

- les gènes ayant une fonction comparable
- les gènes codant pour des protéines associées à la protéine codée par le gène YLR087c
- les gènes codant pour des protéines ayant des similarités de séquences, c'est-à-dire au moins 50% d'homologie, de préférence au moins 60% d'homologie, et plus préférentiellement au moins 70% d'homologie avec la protéine codée par le gène YLR087c.

**EXEMPLE 5**

**Test panification en pousse bloquée**

Les levures de panification fraîches obtenues selon l'exemple 2 avec les souches S47 et S47-12b1 sont testées selon la formule et le schéma de panification suivants:

Formule :

- farine 100 (ou 8000 g)
- eau 61 (ou 4880 g)
- sel 2,1 (ou 168 g)
- levure fraîche 3 (ou 240 g)
- Croustiliss® détente 1 (ou 80 g)
- gluten 1 (ou 80 g).

Croustiliss® détente est la marque déposée d'un améliorant de panification commercialisé par la Société Lesaffre Ingredients, 26 rue Gabriel Péri, 59700 Marcq-en-Baroeul, France et apportant des mono- et diglycérides d'acides gras (émulsifiant E471), des levures désactivées à pouvoir réducteur, de l'acide ascorbique et des alpha-amylases fongiques à effet secondaire.
La farine est une farine de type 55 donnant à l'alvéographe Chopin :
W = 256 - P = 79 - L = 92 - P/L = 0,86
et ne contenant pas d'acide ascorbique. Ces caractéristiques des farines sont définies dans l'ouvrage: "Guide Pratique d'Analyses dans les Industries des Céréales", coordonnateurs B. GODON et W. LOISEL, Technique et Documentation LAVOISIER, 1984.
Schéma de panification :

- pétrissage pétrin spirale KEMPER® 3 minutes en 1ère vitesse +5 minutes en 2ème vitesse
- température de pâte 25°C
- repos 10 minutes
- division en pâtons de 350 g
- détente 15 minutes
- façonnage sous forme baguette
- apprêt à 28°C à volume constant environ 110 minutes
- blocage des pâtons en fin d'apprêt à 8°C pendant 4 heures, 19 heures et 22 heures
- cuisson après blocage à 8°C, étant entendu qu'après les blocages pendant 19 heures et 22 heures, un temps de réchauffement des pâtons d'une heure à 28°C est observé.

Le pétrin utilisé est un pétrin KEMPER® de type "Mixhneter Standard" fabriqué par EMIL KEMPER GMBH 4835 Rietberg 2 - Neueckirchen - Allemagne.
Résultats :
Les baguettes obtenues avec la levure de panification issue de la souche S47 sont trop développées dès 4 heures à 8°C, avec des coups de lame peu jetés. Ces défauts sont accentués après 19 heures et 22 heures. Les baguettes obtenues avec la levure de panification issue de la souche S47-12b1 sont bien développées, avec des coups de lame bien jetés et un bel aspect extérieur après les blocages de 4 heures, 19 heures et 22 heures.

**EXEMPLE 6**

**Test panification en pousse lente**

Les levures de panification fraîches obtenues selon l'exemple 2 avec les souches S47 et S47-12b1 sont testées selon la formule et le schéma de panification suivants:

Formule :

- farine 100 (soit 8000 g)
- eau 61 (soit 4880 g)
- sel 2,1 (soit 168 g)

- levure fraîche 1 (soit 80 g)
- Croustiliss® pousse lente 1 (soit 80 g)
- gluten 0,5 (soit 40 g).

La farine est du même type que dans l'exemple précédent.
Croustiliss® pousse lente est la marque déposée d'un améliorant de panification commercialisé par Lesaffre Ingredients et apportant des mono- et diglycérides d'acides gras, de l'acide ascorbique et des alpha-amylases fongiques dans les qualités et doses optimales pour les procédés de type pousse lente.
Schéma de panification :

- pétrissage pétrin spirale KEMPER® 3 minutes en 1ère vitesse
+5 minutes en 2ème vitesse
- température de pâte 24°C
- repos 15 minutes
- division en pâtons de 350 g
- détente 15 minutes
- façonnage sous forme baguette
- apprêt de 16 heures à 24 heures à 15°C puis cuisson.

Résultats :
Les baguettes obtenues avec la levure de panification issue de la souche S47 sont trop levées au bout de 16 heures, elles sont trop volumineuses, les coups de lame sont peu jetés. On a déjà dépassé ou on est en limite du temps de fermentation maximum pour cette levure.

Par contre dans la plage 16 à 24 heures, on obtient avec la levure de panification obtenue avec la souche S47-12b1 des baguettes avec un bel aspect extérieur, avec des coups de lame nets. Au bout de 16 heures, les baguettes sont un peu moins développées, au bout de 24 heures, on obtient des baguettes en limite supérieure de volume. Pendant 8 heures, on a avec une pâte pétrie et façonnée la veille, des pâtons prêts à cuire à la demande, et donnant des baguettes ayant bel aspect.
Bien entendu, ces exemples ne sont pas limitatifs. Par exemple, il est clair qu'avec la nouvelle levure de panification obtenue avec la souche S47-12b1, on peut coupler pousse lente et pousse bloquée afin d'allonger la plage de cuisson. Elle permet d'obtenir aisément des pâtons prêts à cuire à la demande sur de longues périodes de temps, au moins 4 heures, de préférence au moins 8 heures.

**<u>EXEMPLE 7</u>**

**Pâtes boulangères fermentées en masse bloquée**

La formule utilisée est une formule standard de pain français caractérisée par une hydratation supérieure de la pâte d'au moins 4 points, en raison du schéma de fabrication avec un pointage ou première fermentation qui est long, ce qui donne à la pâte une force importante.
Cette formule est :

- farine 100
- eau 66
- sel 2
- levure fraîche 3
- améliorant 1

Les levures fraîches utilisées sont les levures obtenues selon l'exemple 2 avec les souches S47 (témoin) et S47-12b1 (souche présentant le phénotype de sensibilité au froid en fermentation).
L'améliorant utilisé peut être l'améliorant Ibis® bleu, ou de préférence un améliorant de la gamme Croustiliss®, améliorants vendus par Lesaffre Ingredients. Plus la pâte en masse sera conservée longtemps plus un améliorant Croustiliss® apportant des monoglycérides d'acides gras et richement dosé en acide ascorbique sera nécessaire.
Le pétrissage est réalisé sur pétrin spirale de la Société VMI, ZI Nord, F-85601 Montaigu, 4 minutes en première vitesse, suivies de 8 minutes en seconde vitesse. La pâte obtenue est à 24°C.
Les pâtons obtenus en bacs de 8 kg sont mis en chambre froide à -10°C jusqu'à ce que la température à coeur de la pâte soit de +4°C, puis conservés à +4°C.

Pendant le temps de descente en température, on a une première fermentation ou pointage.

Après 3 jours de blocage à 4°C, la pâte avec la levure témoin est trop fermentée, alors que la pâte avec la levure sensible au froid (souche S47-12b1) reste stable. La pâte témoin n'est guère tolérante à l'apprêt. Après 7 jours de blocage, la pâte témoin n'est plus utilisable. Dans toutes les fabrications de pains comportant les opérations complémentaires de division de la pâte, mise en boule, façonnage de la pâte, apprêt court, cuisson, on obtient pendant les 7 jours avec la pâte réalisée avec la levure obtenue avec la souche S47-12b1 des baguettes à l'ancienne d'excellente qualité organoleptique. Ces opérations complémentaires, compte tenu de l'emploi d'une pâte fermentée ayant subi un pointage long donnant force et arôme, peuvent être réalisées en moins de 3 heures, de préférence en moins de 2 heures. Ce schéma de fabrication avec emploi d'une levure de panification obtenue avec une souche ayant un phénotype de sensibilité au froid en fermentation a les avantages suivants:

- production de pâte en masse en grande quantité se conservant sans problème pendant au moins 7 jours,
- réglage de la température de refroidissement de manière à avoir une première fermentation ou pointage donnant force et arôme à la pâte, et conservant ensuite ses propriétés rhéologiques pendant au moins 7 jours,
- obtention d'un pain de très haute qualité organoleptique avec un schéma de fabrication final court de durée maximale 3 heures et de préférence moins de 2 heures.

De manière générale, l'emploi de levures obtenues avec une souche ayant un phénotype de sensibilité au froid en fermentation permet d'obtenir des pâtes en masse quelle que soit leur composition se conservant bien à 4°C, faciles à transporter. Ces pâtes en masse conditionnées dans des grands conditionnements, par exemple dans des seaux, ou dans des bacs (ce qui est équivalent au vrac) présentent de grands avantages au niveau centralisation ou regroupement des fabrications, possibilités de distribution de la pâte en masse sur de nombreux points de fabrication finale. Cet emploi de levures ayant un phénotype de sensibilité au froid est notamment intéressant pour préparer des pâtes prêtes à l'emploi ou à la cuisson pour distribution dans les différents points de cuisson.

Ces avantages logistiques sont obtenus en général quel que soit le moment où le processus de fabrication est arrêté par des températures inférieures ou égales à 10°C : levain, pâte fermentée en masse, pâte façonnée, pâte prête à cuire.

De plus ces avantages peuvent être couplés avec le choix de procédés de fabrication donnant des avantages importants, supplémentaires dans la fabrication du pain:

- l'utilisation de pâtes boulangères pour pains de type français en masse bloquée dont le refroidissement est réglé de manière à avoir une première fermentation longue ou un pointage long est un exemple de ces avantages. Il permet ensuite rapidement d'obtenir des pains à l'ancienne très aromatiques;
- le réglage de la composition et des arômes produits dans les levains contenant une flore composée d'une souche de levure sensible au froid et de bactéries lactiques de préférence psychrophiles, c'est-à-dire se développant rapidement au froid, est un autre exemple d'application nouvelle particulièrement intéressante en panification.

## Revendications

1. Souche de levure de panification **caractérisée par le fait qu'**elle permet d'obtenir des levures fraîches de panification:

    - donnant au moins 100 ml de $CO_2$ en 2 heures à 30°C dans le test $A_1$, de préférence au moins 110 ml de $CO_2$, de préférence encore au moins 150 ml de $CO_2$,
    - obéissant au rapport suivant :

$$\frac{\text{dégagement } CO_2 \text{ en 48 heures à 8°C dans le test } A_1}{\text{dégagement } CO_2 \text{ en 2 heures à 30°C dans le test } A_1} \text{ inférieur à } 0{,}45,$$

de préférence inférieur à 0,40 et de préférence encore inférieur à 0,30 étant entendu que, selon le test $A_1$, à 20 g de farine incubée à la température choisie pour la mesure, on ajoute un poids de levure comprimée correspondant à 160 mg de matières sèches, cette levure étant délayée dans 15 ml d'eau contenant 27 g de NaCl par litre et 4 g de $(NH_4)_2SO_4$ par litre ; on malaxe à l'aide d'une spatule pendant 40 secondes, de manière à obtenir une pâte que l'on place au bain-marie réglé à la température choisie ; treize minutes après le début du malaxage, le récipient contenant la pâte est fermé hermétiquement; la quantité totale de gaz produit est mesurée après 60, puis 120 minutes ou plusieurs heures; cette quantité étant exprimée en ml à 20°C et sous 760 mm Hg.

**2.** Souche S47-12b1 déposée au CNCM sous le n° I-1645.

**3.** Souche L30-13 déposée au CNCM sous le n° I-1647.

**4.** Souche L30-91 déposée au CNCM sous le n° I-1646.

**5.** Souche HL13.2.30 déposée au CNCM sous le n° I-1841.

**6.** Souche **caractérisée en ce qu'**elle possède le même phénotype que les souches selon l'une des revendications 1 à 5 et qu'elle porte une mutation non létale sur tous ses gènes YLR087c, ladite mutation étant un codon stop.

**7.** Procédé d'obtention d'une souche selon la revendication 1 comprenant la transformation d'une souche industrielle de panification consistant à rendre conditionnelle l'expression de l'un de ceux des gènes de cette souche qui ont une action directe ou indirecte sur la fermentation des sucres en fonction de la température en ayant recours à un promoteur dont l'action sur l'expression dudit gène dépend de la température

**8.** Utilisation des levures fraîches ou sèches obtenues avec une souche selon l'une des revendications 1 à 6 dans un procédé de panification différée.

**9.** Utilisation suivant la revendication 8, dans laquelle le procédé de panification différée est un procédé de pousse lente où l'apprêt est réalisé à des températures entre 10 et 18°C, de préférence à environ 15°C, pendant 15 à 24 heures.

**10.** Utilisation suivant la revendication 8, dans laquelle le procédé de panification différée est un procédé de pousse bloquée où la fermentation est bloquée après l'apprêt, juste avant la mise au four.

**11.** Utilisation des levures fraîches ou sèches obtenues avec une souche selon l'une des revendications 1 à 6 dans un procédé de panification différée conduisant à des pâtons qui restent prêts à cuire pendant une période d'au moins 4 heures, et de préférence pendant au moins 8 heures.

**12.** Utilisation suivant l'une quelconque des revendications 8 à 11, dans laquelle le procédé de panification différé conduit à un pain de type français.

**13.** Utilisation des levures fraîches ou sèches obtenues avec une souche selon l'une des revendications 1 à 6 pour l'obtention de pâtes en masse.

**14.** Utilisation suivant la revendication 13, selon laquelle les pâtes en masse sont des pâtes préfermentées, des levains ou des pâtes de première fermentation.

**15.** Utilisation selon la revendication 13 selon laquelle les pâtes en masse sont des pâtes jaunes fermentées.

**16.** Utilisation de levures fraîches ou sèches obtenues avec une souche selon l'une des revendications 1 à 6 pour la production de pâtes fermentées en masse correspondant à une longue préfermentation, permettant ensuite d'obtenir avec un apprêt court des pains aromatiques de type français.

**17.** Utilisation des levures obtenues avec une souche selon l'une des revendications 1 à 6 pour l'obtention de levains de'bactéries et de levures.

## Claims

**1.** Bread-making yeast strain caracterized by the fact that it enables the obtention of fresh bread-making yeasts:

- giving at least 100 ml of $CO_2$ in two hours at 30°C in the test $A_1$, preferably at least 110 ml of $CO_2$, still preferably at least 150 ml of $CO_2$.
- corresponding to the following ratio :

$$\frac{\text{Release of } CO_2 \text{ in 48 hours at } 8°C \text{ in test } A_1}{\text{Release of } CO_2 \text{ in 2 hours at } 30°C \text{ in test } A_1}$$

lower than 0.45, preferably lower than 0.40 and still preferably lower than 0.30
it being understood that according to text $A_1$, there is added to 20 g of flower incubated at the temperature selected for the measure a weight of compressed yeast corresponding to 160 mg of dry matter, the said yeast being diluted in 15 ml of water containing 27 g of NaCl per litre and 4 g of $(NH_4)_2SO_4$ per litre; this is mixed with a spatula during 40 seconds in order to obtain a dough which is put in a water-bath or bain-marie regulated at the selected temperature ; thirteen minutes after the start of the mixing, the vessel containing the dough is hermetically closed; the total amount of gas which is produced is measured after 60, then 120 minutes or several hours; that quantity being expressed in ml at 20°C and under 760 mm of Hg.

**2.** Strain S47-12b1 deposited at the CNCM under the number I-1645.

**3.** Strain L30-13 deposited at the CNCM under the number I-1647.

**4.** Strain L30-91 deposited at the CNCM under the number I-1646.

**5.** Strain HL13.2.30 deposited at the CNCM under the number I-1841.

**6.** Strain **characterized by** the fact that it has the same phenotype as the strains according to one of claims 1 to 5 and that it carries non-lethal mutation on all its genes YLR087c, the said mutation being a stop codon.

**7.** Process for the obtention of a strain according to claim 1, comprising the transformation of an industrial bread-making strain, transformation consisting to make conditional the expression of one of those of the genes of said strain which have a direct or indirect action on the fermentation of the sugars in function of the temperature, having recourse to a promoter whose action on the expression of the said gene depends from the temperature.

**8.** Use of the fresh or dry yeasts obtained starting from a strain according to one of claims 1 to 6 in a process of deferred bread-making.

**9.** Use according to claim 8 wherein the deferred bread-making process is a slow-rise method wherein finishing is carried out at temperatures between 10 and 18°C, preferably at about 15°C during 15 to 24 hours.

**10.** Use according to claim 8 wherein the differed bread-making process is a blocked-rise method wherein the fermentation is blocked after finishing just before placing into the oven.

**11.** Use of the fresh or dry yeasts obtained using a strain according to one of claims 1 to 6 in a deferred bread-making process providing doughs which remain ready to be cooked during a period of at least 4 hours and preferably during at least 8 hours.

**12.** Use according to anyone of claims 8 to 11, wherein the deferred bread-making process provides a French type bread.

**13.** Use of the fresh or dry yeasts obtained using a strain according to one of claims 1 to 6 for the obtention of a bulk dough.

**14.** Use according to claim 13, wherein the bulk doughs are pre-fermented doughs, leavens or doughs of first fermentation.

**15.** Use according to claim 13, wherein the bulk doughs are yellow fermented doughs.

**16.** Use of fresh or dry yeasts obtained using a strain according to one of claims 1 to 6 for the production of fermented bulk doughs corresponding to a long pre-fermentation, enabling then the obtention with a short finishing of aromatic breads of the French type.

**17.** Use of the yeasts obtained using a strain according to one of claims 1 to 6, for the obtention of leavens of bacteria and of yeasts.

**Patentansprüche**

**1.** Brotbereitungshefestamm, **dadurch gekennzeichnet, dass** er ermöglicht, frische Brotbereitungshefen zu erhalten, welche:

- im Test $A_1$ in 2 Stunden bei 30°C mindestens 100 ml $CO_2$, bevorzugt mindestens 110 ml $CO_2$, noch stärker bevorzugt mindestens 150 ml $CO_2$ ergeben,
- das nachfolgende Verhältnis erfüllen:

$$\frac{CO_2\text{-Entwicklung innerhalb von 48 Stunden bei 8°C im Test } A_1}{CO_2\text{-Entwicklung innerhalb von 2 Stunden bei 30°C im Test } A_1}$$

kleiner als 0,45, bevorzugt kleiner als 0,40 und noch stärker bevorzugt kleiner als 0,30,

wobei verstanden wird, dass gemäß Test A1 man zu 20 g Mehl, inkubiert bei der für die Messung ausgewählten Temperatur, eine Menge an Presshefe zugibt, die 160 mg Trockenmasse entspricht, diese Hefe in 15 ml Wasser, enthaltend 27 g NaCl pro Liter und 4 g $(NH_4)_2SO_4$ pro Liter, verdünnt, mit Hilfe eines Spatels 40 Sekunden derart mischt, so dass man eine Paste erhält, welche man auf das bei der ausgewählten Temperatur eingestellte Wasserbad stellt; dreizehn Minuten nach dem Beginn des Mischens den die Paste enthaltenden Behälter luftdicht verschließt; die Gesamtmenge des erzeugten Gases nach 60, danach 120 Minuten oder mehreren Stunden misst; wobei diese Menge in ml bei 20°C und unter 760 mm Hg angegeben wird.

**2.** Stamm S47-12b1, hinterlegt beim CNCM unter der Nummer I-1645.

**3.** Stamm L30-13, hinterlegt beim CNCM unter der Nummer I-1647.

**4.** Stamm L30-91, hinterlegt beim CNCM unter der Nummer I-1646.

**5.** Stamm HL13.2.30, hinterlegt beim CNCM unter der Nummer 1-1841.

**6.** Stamm, **dadurch gekennzeichnet, dass** er den gleichen Phänotyp hat wie die Stämme nach einem der Ansprüche 1 bis 5, und dass er eine nicht-letale Mutation in allen seinen YLR087c Genen aufweist, wobei die Mutation ein Stopcodon ist.

**7.** Verfahren um einen Stamm nach Anspruch 1 zu erhalten, umfassend das Transformieren eines industriellen Brotbereitungsstammes, was darin besteht, die Expression eines derjenigen Gene dieses Stamms, welches eine direkte oder indirekte Wirkung auf die temperaturabhängige Fermentierung von Zuckern hat, konditionell zu machen, mit Hilfe eines Promotors, dessen Wirkung auf die Funktion des Gens von der Temperatur abhängt.

**8.** Verwendung von Frischhefen oder Trockenhefen, welche mit einem Stamm nach einem der Ansprüche 1 bis 6 erhalten wurden, in einem aufgeschobenen Brotbereitungsverfahren.

**9.** Verwendung nach Anspruch 8, wobei das aufgeschobene Brotbereitungsverfahren ein Verfahren mit langsamem Gehen Lassen ist, wobei die Zubereitung bei Temperaturen zwischen 10 und 18°C, bevorzugt bei etwa 15°C während 15 bis 24 Stunden durchgeführt wird.

**10.** Verwendung nach Anspruch 8, wobei das aufgeschobene Brotbereitungsverfahren ein Verfahren mit blockiertem Gehen Lassen ist, wobei die Fermentation nach der Zubereitung, direkt vor dem Einbringen in den Ofen, blockiert ist.

**11.** Verwendung von Frischhefen oder Trockenhefen, welche mit einem Stamm nach einem der Ansprüche 1 bis 6 erhalten wurden, in einem aufgeschobenen Brotbereitungsverfahren, das zu Teigwürsten führt, die während eines Zeitraums vom mindestens 4 Stunden und bevorzugt während mindestens 8 Stunden backfertig bleiben.

**12.** Verwendung nach einem der Ansprüche 8 bis 11, wobei das aufgeschobene Brotbereitungsverfahren zu einem Brot nach französischer Art führt.

**13.** Verwendung von Frischhefen oder Trockenhefen, welche mit einem Stamm nach einem der Ansprüche 1 bis 6 erhalten wurden, um Teigmassen zu erhalten.

**14.** Verwendung nach Anspruch 13, wobei die Teigmassen präfermentierte Teige, Sauerteige oder Teige erster Fermentation sind.

**15.** Verwendung nach Anspruch 13, wobei die Teigmassen gelbe fermentierte Teige sind.

**16.** Verwendung von Frischhefen oder Trockenhefen, welche mit einem Stamm nach einem der Ansprüche 1 bis 6 erhalten wurden, zur Herstellung von fermentierten Teigmassen, die einer langen Präfermentation entsprechen, wodurch danach aromatische Brote nach französischer Art mit einer kurzen Zubereitung erhalten werden können.

**17.** Verwendung von Hefen, welche mit einem Stamm nach einem der Ansprüche 1 bis 6 erhalten wurden, um Bakterienfermente und Hefen zu erhalten.

FIG.1.

CONSTRUCTION DU PLASMIDE
YIp lac 211-ura::PH^R

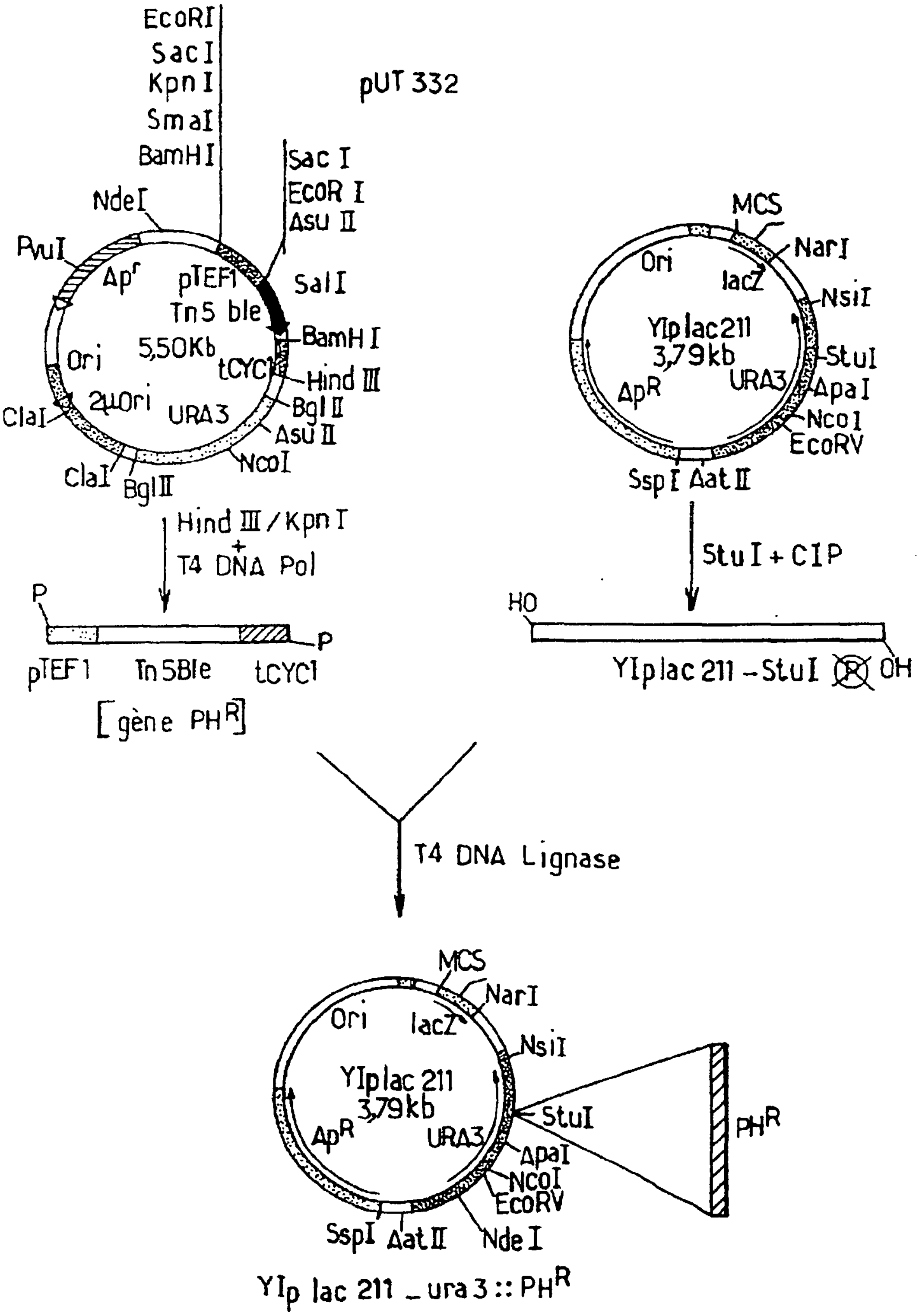


YIp lac 211 _ ura3 :: PH^R

FIG.2.

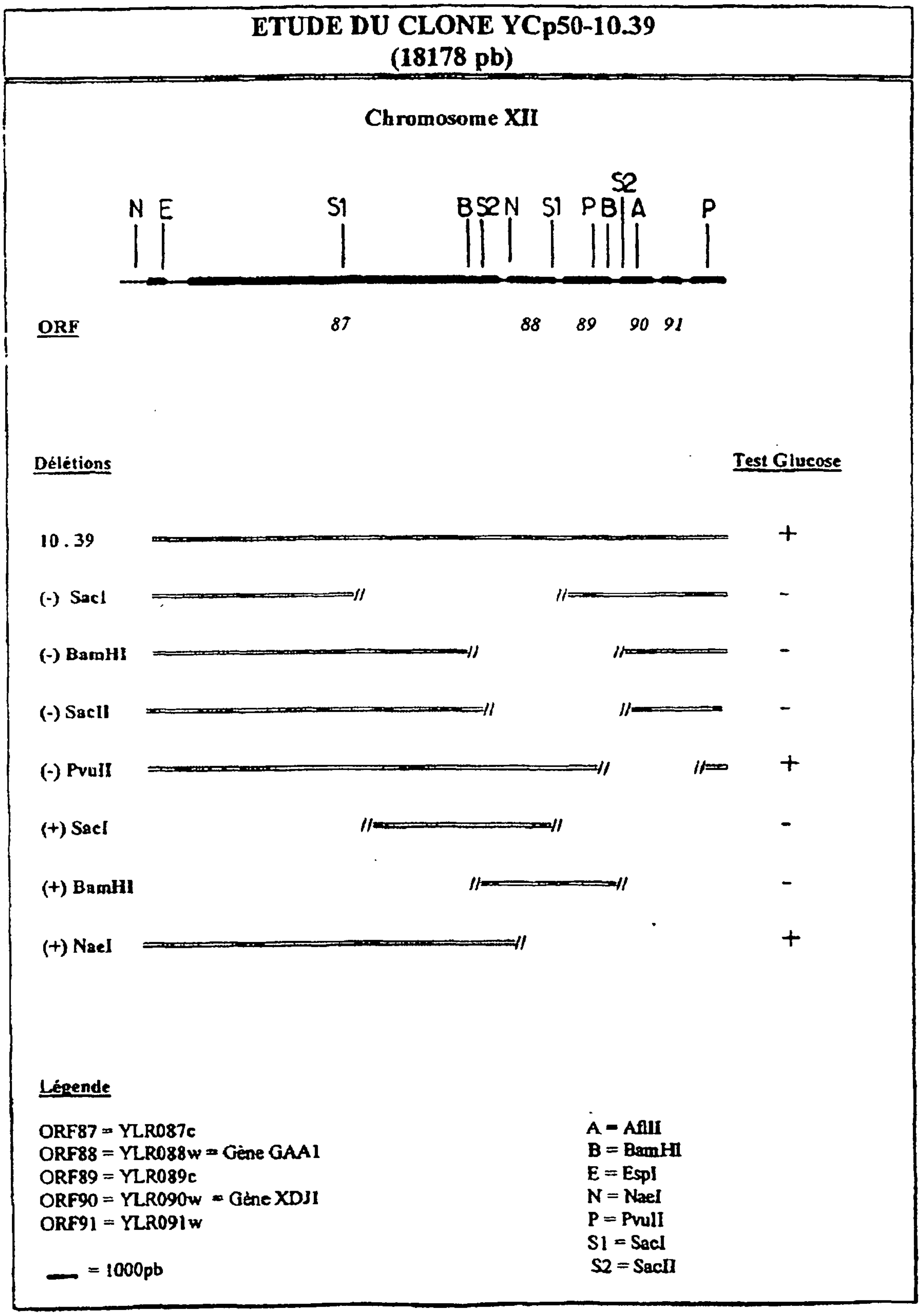
ETUDE DU CLONE YCp50-10.39
(18178 pb)
Chromosome XII
N E S1 B S2 N S1 P B S2 A P
ORF
87 88 89 90 91
Délétions
Test Glucose
10.39 +
(-) SacI -
(-) BamHI -
(-) SacII -
(-) PvuII +
(+) SacI -
(+) BamHI -
(+) NaeI +
Légende
ORF87 = YLR087c
ORF88 = YLR088w = Gène GAA1
ORF89 = YLR089c
ORF90 = YLR090w = Gène XDJ1
ORF91 = YLR091w
= 1000pb
A = AflII
B = BamHI
E = EspI
N = NaeI
P = PvuII
S1 = SacI
S2 = SacII

FIG.3.

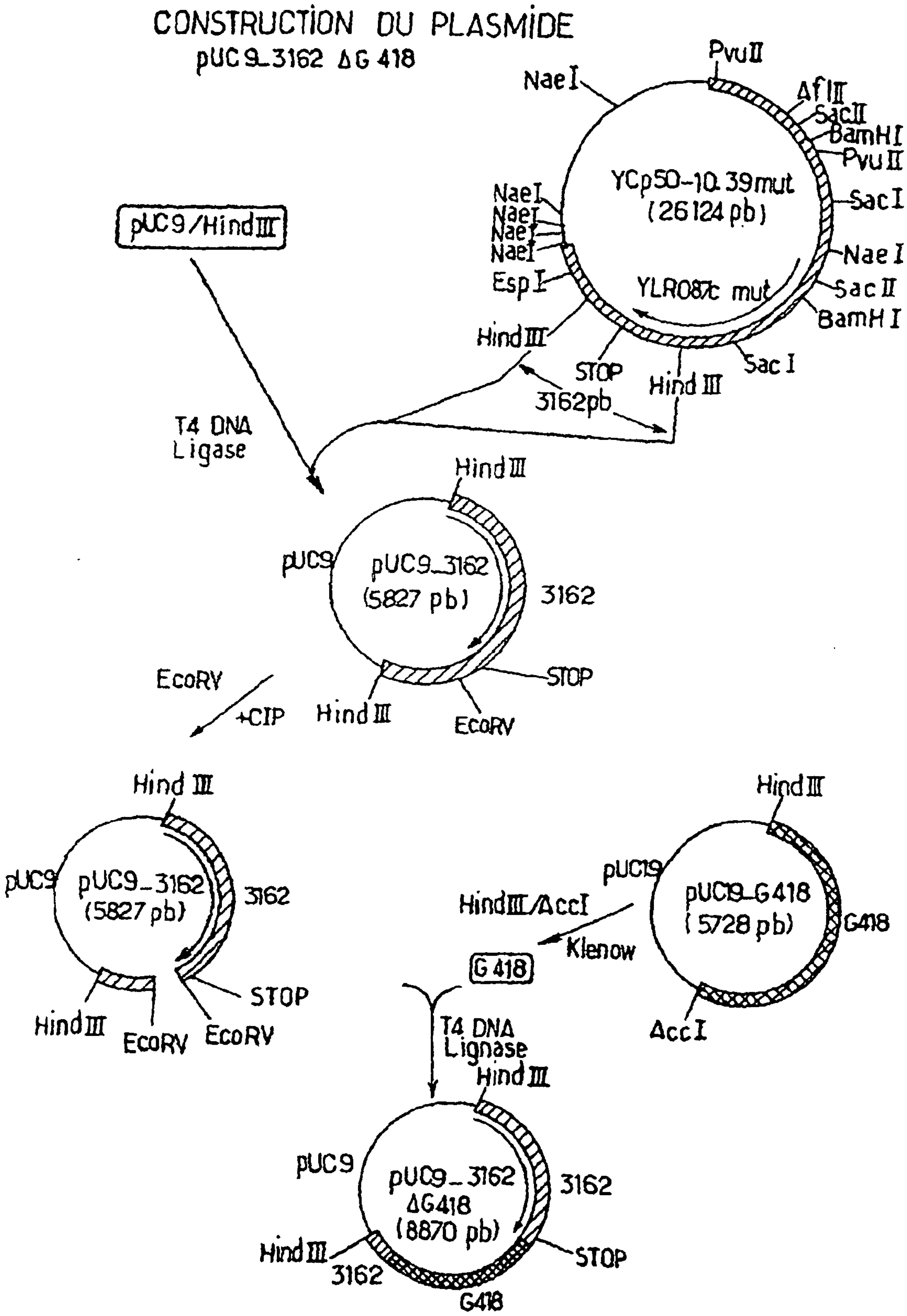
CONSTRUCTION DU PLASMIDE
pUC9_3162 ΔG 418
pUC9/HindIII
T4 DNA Ligase
YCp50-10.39mut (26124 pb)
YLR087c mut
PvuII
AflII
SacII
BamHI
PvuII
SacI
NaeI
SacII
BamHI
SacI
HindIII
STOP
NaeI
EspI
3162pb
pUC9_3162 (5827 pb)
pUC9
3162
STOP
HindIII
EcoRV
EcoRV
+CIP
HindIII/AccI
Klenow
G418
pUC19
pUC19_G418 (5728 pb)
G418
AccI
T4 DNA Lignase
pUC9_3162 ΔG418 (8870 pb)
3162
G418

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 487878 A
- EP 663441 A
- WO 9301724 A
- WO 9419955 A
- EP 667099 A
- EP 556905 A
- EP 0818535 A
- EP 92401168 A
- EP 0511108 A
- US 4396632 A
- FR 9601562 B


### Littérature non-brevet citée dans la description


- **BURROWS ; HARRISON.** *Journal of the Institute of Brewing,* Janvier 1959, vol. LXV (1
- **LITTLEWOOD, B.S. ; DAVIES, J.E.** Enrichment for temperature sensitive and auxotrophic mutants in Saccharomyces cerevisiae by Tritium suicide. *Mutation Research,* 1973, vol. 17, 315-322
- **BOUCHERIE H. et al.** S. cerevisiae 2D protein map. *Yeast,* 1995, vol. 11 (7), 601-613
- **STRAUSS, E.J. ; GUTHRIE, C.** *Genes and Development,* 1991, vol. 5, 629-641
- **WICKNER, R.B. et al.** *Yeast,* 1987, vol. 3, 51-57
- **YAMANO, S. et al.** *Mol.Gen.Genet.,* 1987, vol. 210, 413-18
- **MUNOS-DORADO J. et al.** Identification of cis-and trans-acting elements involved in the expression of cold shock inducible TIP1 gene of Yeast Saccharomyces cerevisiae. *Nucleic Acid Research,* 1994, vol. 22 (4), 560-568
- **KOWALSKI, L. et al.** Cold shock induction of a family of TIP1 related proteins associated with the membrane in Saccharomyces cerevisiae. *Molecular Microbiology,* Janvier 1995, vol. 15 (2), 341-353
- **NEHLIN, J.O. ; RONNE H.** *EMBO J.,* 1990, vol. 9, 2891-2898
- **YOSHIMATSU ET T. ; VAGANA F.** Control of Gene Expression by Artificial Introns in Saccharomyces cerevisiae. *Science,* 1988, vol. 244 (4910), 1346-1348
- **ROSE, M.D. et al.** *Gene,* 1987, vol. 60, 237-243
- **J. SAMBROOK ; E.F. FRITSCH ; T. MANIATIS.** MOLECULAR CLONING. Cold Spring Harbor Laboratory Press, 1989
- **GIETZ, R.D. ; SUGINO, A.** *Gene,* 1988, vol. 74, 527-534
- **GATIGNOL, A. et al.** *Mol. Gen. Genet.,* 1987, vol. 207, 342-348
- **GIETZ, D. et al.** *Nucl. Acids Res.,* 1992, vol. 20, 1425
- **NESS et al.** *J.Sci.Food Agric.,* 1993, vol. 62, 89-94
- **GIETZ, R.D. ; SUGINO, A.** *Gene,* 1988, vol. 74, 527-534
- **IWASAKI, T. et al.** *Gene,* 1991, vol. 109, 81-87
- **ORR-WEAVER, T.L et al.** *Methods Enzymol.,* 1983, vol. 101, 228-245
- **RODSTEIN, R.** *Methods in Enzymology,* 1991, vol. 194, 281-301
- **MEILHOC, E et al.** *Biotechnology,* 1990, vol. 8, 223-227
- **SCHAAFF et al.** *Curr.Genet.,* 1989, vol. 15, 75-81
- **BOY-MARCOTTE, E. ; JACQUET, M.** *Gene,* 1982, vol. 20, 433-440
- **ALANI, E. et al.** *Genetics,* 1987, vol. 116, 541-545

LISTE DE SEQUENCES

(1) INFORMATIONS GENERALES:

(i) DEPOSANT:

(A) NOM: LESAFFRE ET CIE
(B) RUE: 41 rue Etienne Marcel
(C) VILLE: PARIS
(E) PAYS: FRANCE
(F) CODE POSTAL: 75001

(ii) TITRE DE L'INVENTION: Nouvelles levures de panification sensibles au froid

(iii) NOMBRE DE SEQUENCES: 2

(iv) LISTE DECHIFFRABLE PAR ORDINATEUR:

(A) TYPE DE SUPPORT: Floppy disk

(B) ORDINATEUR: IBP PC compatible
(C) SYSTEME D'EXPLOITATION: PC-DOS/MS-DOS
(D) LOGICIEL: PatentIn Release #1.0, Version #1.30 (OEB)

(vi) DONNEES RELATIVES A LA DEMANDE ANTERIEURE:

(A) NUMERO DE DEPOT: FR 96 01562
(B) DATE DE DEPOT: 08-FEV-1996
(C) CLASSEMENT: A21D C12N

(vii) INFORMATIONS CONCERNANT LE CONSEIL OU LE MANDATAIRE:

(A) NOM: KOCH Gustave
(C) NUMERO DE REFERENCE: DPE970018/GK

(ix) INFORMATIONS CONCERNANT LES TELECOMMUNICATIONS:

(A) TELEPHONE: 01-44-63-41-11
(B) TELECOPIEUR: 01-42-80-01-59
(C) E-MAIL: info@plass.com

(2) INFORMATIONS CONCERNANT LA SEQ ID No. 1:

(i) CARACTERISTIQUES DE LA SEQUENCE:

(A) LONGUEUR: 9621 paires de bases
(B) TYPE: acide nucléique
(C) NOMBRE DE BRINS: simple
(D) CONFIGURATION: linéaire

(ii) TYPE DE MOLECULE: ADN génomique

(iii) HYPOTHETIQUE: NON

(iv) ANTI-SENS: NON, la séquence présentée est écrite dans le sens de traduction de la protéine.

(vi) ORIGINE:

(A) ORGANISME: Saccharomyces cerevisiae
(B) SOUCHE: HL13.2.30, souche déposée au CNCM sous le numéro I-1841

(viii) POSITION DANS LE GENOME:

(A) CHROMOSOME: XII
(B) COORDONNEES: Nucléotides 306483 à 316106 (brin Crick)
(C) SOURCE IMMEDIATE:
CLONE: DH5α[YCp50-10.39mut], souche déposée au CNCM sous le n° I-1842

(ix) CARACTERISTIQUE ADDITIONNELLE:

(A) NOM/CLE: gène YLR087c muté
(B) EMPLACEMENT: 1...9621
(C) METHODE D'IDENTIFICATION: par similitude avec séquences présentes dans les bases de données d'acides nucléiques

(ix) CARACTERISTIQUE ADDITIONNELLE:

(A) NOM/CLE: séquence codante

(B) EMPLACEMENT: 376...7863

(ix) CARACTERISTIQUE ADDITIONNELLE:

(A) NOM/CLE: mutation
(B) EMPLACEMENT: 7865
(C) AUTRE INFORMATION: codon STOP (TGA)

(xi) DESCRIPTION DE LA SEQ ID No. 1:

```
TCTTGGTGTA TTATACGCGT CTTTGGTCTG TCAAGTCCTT TTATAAGACA GCAAATGTCA 60
TTTCTTTCTA AGTGTAGCCG GCGTGTATTG TTTATGACTT TTTTTTCTAC CGCTGGCATT 120
AAAGTGAGAG GTTCACGATG CGTTCCCAAT AACTTCGTAG GGATGGCGAT CAATATACAA 180
ATAGAGGGAA GCCAAACTCC CCACTATAAA GCTGTTGGCA CGGTTTTATA AGCTTCAACC 240
TTTCTAGGCT ACAGGATACT CAAAAAAAAT TACTGCGGTA TTATCAACAT AGTGCTCTTT 300
GGAAAAAAAA AAAGAATAGT ACTCATTAAA GCCACCTGAC TCAAGTCTTC TATTGACGGT 360
AATAAGTTAG CAAGCATGGA AGCTATTTCA CAATTACGTG GTGTTCCATT GACACACCAA 420
AAGGACTTTA GCTGGGTCTT TTTAGTAGAT TGGATTCTCA CGGTGGTAGT ATGTTTGACA 480
ATGATATTCT ACATGGGAAG AATCTATGCA TACCTTGTAA GTTTTATATT AGAATGGCTA 540
CTATGGAAAC GAGCGAAAAT CAAGATAAAT GTTGAGACAC TTCGTGTCTC CTTACTAGGT 600
GGTCGAATAC ATTTTAAAAA CCTTTCCGTA ATACACAAAG ATTATACGAT TTCGGTATTA 660
GAGGGTAGTT TAACATGGAA ATACTGGCTT TTAAATTGCA GAAAAGCAGA ATTGATAGAG 720
AATGACAAGT CTTCTTCTGG CAAAAAAGCA AAGCTTCCGT GTAAAATTTC CGTAGAATGT 780
GAAGGTCTAG AAATTTTTAT TTACAACAGA ACAGTGGCGT ACGATAATGT TATAAACTTA 840
CTATCAAAAG ATGAACGCGA TAAATTTGAA AAATACCTTA ATGAGCATTC TTTTCCTGAA 900
CCTTTTAGCG ATGGAAGTAG TGCTGATAAA TTAGATGAAG ATCTAAGCGA ATCTGCATAC 960
ACAACGAACT CTGATGCATC AATTGTTAAT GACAGGGACT ACCAAGAAAC AGATATCGGC 1020
AAACATCCAA AGCTACTGAT GTTTTTACCA ATTGAGCTTA AATTTAGCCG CGGTTCCCTA 1080
CTGTTAGGAA ACAAATTCAC GCCATCTGTT ATGATTCTAA GTTATGAAAG TGGAAAAGGC 1140
ATAATAGATG TTTTACCTCC AAAAGAGCGA TTAGATTTAT ACAGAAATAA AACACAGATG 1200
GAATTCAAAA ACTTCGAAAT TTCTATCAAA CAAAATATTG GTTACGATGA TGCTATTGGA 1260
TTGAAGTTTA AAATAGATAG AGGGAAAGTG TCAAAGTTAT GGAAAACGTT TGTACGAGTC 1320
TTTCAGATAG TAACCAAGCC TGTTGTACCG AAAAAGACTA AAAAAAGCGC AGGCACATCA 1380
GATGACAATT TCTATCATAA ATGGAAAGGT TTATCTCTTT ATAAGGCTTC TGCGGGCGAC 1440
GCTAAAGCAA GTGATTTAGA TGATGTTGAG TTCGATTTGA CGAACCATGA ATATGCTAAA 1500
TTTACATCAA TTTTAAAATG CCCAAAGGTC ACAATTGCAT ATGACGTGGA TGTTCCGGGC 1560
GTTGTGCCAC ATGGTGCACA TCCGACAATA CCTGATATTG ATGGACCAGA TGTGGGCAAT 1620
AACGGAGCAC CTCCAGATTT TGCTTTAGAT GTTCAAATTC ACGGAGGATC CATCTGTTAC 1680
GGACCTTGGG CTCAAAGACA AGTCAGTCAT CTACAAAGAG TTCTATCACC GGTAGTTTCA 1740
AGGACAGCCA AACCTATAAA AAAACTCCCG CCAGGTTCTA GAAGAATATA TACACTTTTC 1800
AGGATGAATA TATCAATAAT GGAAGATACT ACTTGGCGTA TACCGACGAG GGAAAGTAGC 1860
AAAGACCCCG AATTTTTGAA ACACTACAAA GAAACTAATG AAGAATATAG GCCATTTGGA 1920
TGGATGGATC TCCGATTTTG TAAGGACACC TATGCRAATT TCAATATAAG TGTTTGTCCT 1980
```

ACAGTGCAAG GTTTTCAGAA TAATTTCCAT GTTCATTTCC TGGAAACCGA AATTAGGTCA 2040
AGTGTTAATC ACGATATTTT GTTAAAAAGC AAGGTATTCG ATATTGATGG GGATATTGGA 2100
TATCCATTGG GTTGGAATAG CAAAGCTATA TGGATAATTA ACATGAAGTC AGAACAATTA 2160
GAGGCGTTTC TGCTACGTGA GCATATAACT TTAGTTGCAG ATACGCTTTC AGACTTTTCC 2220
GCTGGTGAYC CTACGCCTTA CGAACTTTTT AGACCATTCG TATACAAAGT CAATTGGGAA 2280
ATGGAAGGAT ATTCYATCTA CTTAAACGTC AATGATCACA ATATTGTTAA CAATCCGTTA 2340
GATTTTAACG AAAACTGTTA TTTATCCCTT CATGGTGATA AGCTTTCAAT TGATGTCACG 2400
GTACCCCGTG AGAGTATTTT GGGGACATAC ACAGATATGT CCTACGAGAT CTCAACTCCA 2460
ATGTTCAGAA TGATGTTAAA TACCCCCCCT TGGAATACAT TGAACGAATT CATGAAACAT 2520
AAAGAAGTGG GGAGAGCATA CGACTTTACA ATTAAAGGTT CTTACCTTCT CTATTCTGAG 2580
TTAGATATTG ATAATGTCGA TACGCTAGTC ATAGAGTGTA ACAGCAAGAG TACAGTACTT 2640
CACTGCTATG GGTTTGTCAT GAGGTATTTA ACAAACGTAA AGATGAATTA CTTCGGTGAA 2700
TTTTTTAATT TTGTGACGTC AGAAGAGTAC ACGGGTGTCC TTGGCGCTAG GGAAGTCGGA 2760
GATGTCACTA CGAAAAGCTC GGTGGCGGAT TTGGCATCTA CTGTAGATTC AGGGTACCAA 2820
AATAGCAGTC TAAAGAACGA ATCTGAGGAT AAAGGTCCTA TGAAAAGGTC AGATTTGAAA 2880
AGGACTACCA ACGAAACTGA TATTTGGTTC ACATTTTCGG TTTGGGATGG TGCTCTGATA 2940
TTACCAGAAA CGATTTACAG TTTTGATCCA TGCATTGCAC TACATTTTGC CGAACTTGTC 3000
GTGGATTTCA GAAGTTGTAA TTATTATATG GACATAATGG CGGTTCTCAA CGGGACTTCA 3060
ATAAAGCGGC ACGTTTCAAA ACAAATAAAT GAAGTATTTG ATTTTATACG TCGTAATAAC 3120
GGTGCTGATG AGCAGGAGCA CGGATTGCTT TCGGACCTCA CCATTCATGG ACATAGAATG 3180
TATGGATTAC CACCCACAGA ACCTACCTAC TTTTGTCAAT GGGATATCAA TCTCGGAGAT 3240
TTATGCATTG ATTCAGATAT TGAATTTATA AAGGGATTCT TTAATTCCTT TTATAAGATA 3300
GGTTTTGGCT ACAATGACTT GGAAAATATA TTATTATATG ACACTGAGAC CATTAATGAT 3360
ATGACCTCGC TAACTGTGCA CGTTGAAAAA ATAAGAATAG GCCTTAAAGA TCCGGTGATG 3420
AAATCTCAAT CAGTTATTAG TGCTGAATCG ATATTGTTTA CTTTGATCGA CTTTGAAAAC 3480
GAAAAATATT CACAAAGAAT AGACGTGAAA ATTCCAAAAT TGACAATTTC GTTGAATTGC 3540
GTGATGGGCG ATGGCGTAGA CACATCATTT CTCAAATTCG AAACAAAATT AAGATTTACA 3600
AACTTTGAGC AATACAAGGA TATCGATAAA AAAAGATCAG AACAACGCAG ATATATAACA 3660
ATACACGATT CACCCTATCA TAGRTGTCCT TTTCTTCTTC CGCTGTTTTA TCAGGATTCG 3720
GATACATACC AAAACCTGTA CGGGGCTATA GCACCATCTT CGTCTATCCC AACTTTACCT 3780
CTTCCCACTT TGCCTGATAC TATAGATTAT ATCATTGAAG ATATTGTGGG CGAGTATGCT 3840
ACCCTTCTGG AGACCACAAA TCCATTCAAG AACATATTCG CAGAAACTCC ATCAACTATG 3900
GAGCCTTCAA GAGCCAGCTT CAGTGAAGAT GATAATGAGG AAGGAGCGGA CCCTTCAAGC 3960
TTCAAACCTG TTGCTTTTAC AGAAGACAGA AACCACGAAA GGGATAACTA TGTTGTTGAT 4020
GTTTCATATA TTCTGTTGGA TGTCGACCCG TTGCTTTTTA TTTTCGCTAA GAGTTTATTA 4080
GAACAGCTTT ACTCTGAAAA CATGGTACAA GTCTTAGACG ATATTGAAAT TGGGATTGTG 4140
AAACGATTAA GCAACCTTCA AGAAGGGATT ACTTCTATTT CAAACATTGA TATCCATATT 4200
GCTTATCTAA ATTTAATTTG GCAAGAGACA GGTGAGGAAG GTTTTGAGCT CTATTTAGAT 4260
CGTATTGATT ATCAAATGAG TGAAAAGTCT CTAGAGAAGA ACCGAACAAA TAAATTATTA 4320
GAGGTAGCAG CTTTAGCAAA GGTAAAAACT GTCAGAGTGA CTGTTAACCA GAAGAAAAAT 4380
CCAGACTTGT CTGAAGATCG TCCCCCTGCA CTGTCGCTAG GGATTGAGGG TTTCGAAGTA 4440
TGGTCTTCTA CAGAAGATAG ACAAGTTAAC TCATTAAACT TAACGTCATC AGATATTACC 4500
ATAGACGAAT CTCAAATGGA ATGGCTGTTT GAGTACTGTA GTGACCAGGG AAATCTTATT 4560
CAAGAGGTTT GCACTTCTTT TAATTCTATT CAGAACACCA GAAGTAATTC AAAGACAGAA 4620
CTCATTTCAA AGCTCACAGC CGCAAGCGAA TATTATCAAA TTAGTCATGA TCCTTACGTC 4680
ATAACAAAAC CTGCTTTTAT TATGAGACTT TCCAAAGGGC ATGTGCGTGA GAATCGTAGT 4740
TGGAAAATTA TTACGCGTCT GAGACACATT TTAACGTACC TTCCTGATGA TTGGCAAAGC 4800
AACATCGACG AAGTGCTAAA AGAAAAGAAA TATACCTCTG CTAAAGATGC AAAAAATATC 4860
TTCATGTCTG TGTTTTCGAC TTGGAGAAAT TGGGAGTTCT CAGATGTTGC AAGGTCGTAT 4920
ATATACGGCA AATTATTCAC GGCAGAAAAT GAGAAACATA AACAAAATTT GATTAAAAAA 4980
TTGTTGAAGT GTACCATGGG ATCATTTTAC CTTACTGTTT ATGGTGAGGG ATATGAGGTT 5040
GAGCATAATT TTGTTGTTGC GGATGCCAAT CTGGTAGTGG ATTTGACGCC TCCGGTGACA 5100
AGCTTACCTT CAAATCGAGA RGAGACTATT GAAATTACGG GAAGAGTAGG CTCAGTAAAA 5160
GGAAAATTCA GTGATAGGTT ACTTAAATTG CAAGATCTTA TTCCACTCAT TGCAGCAGTG 5220
GGCGAAGATG ACAAAAGTGA TCCAAAAAAG GAGTTATCAA AGCAATTCAA AATGAACACC 5280
GTTTTATTAG TGGATAAAAG TGAACTGCAA CTGGTCATGG ACCAAACGAA GCTGATAAGT 5340
AGAACAGTTG GGGGTAGAGT TAGTTTACTA TGGGAAAATC TAAAAGATTC AACTAGTCAA 5400
GCGGGTTCAT TGGTTATATT TTCCCAGAAA TCGGAAGTGT GGTTAAAACA CACATCTGTC 5460
ATTTTGGGAG AAGCTCAACT GCGCGACTTT TCAATTTTAG CGACTACTGA GGCATGGTCA 5520

CACAAGCCTA CGATTCTGAT AAACAACCAG TGCGCAGATC TTCATTTTAG AGCAATGAGT 5580
TCAACTGAGC AATTAGTAAC CGCTATTACT GAAATTAGGG AAAGTCTGAT GATGATTAAA 5640
GAGCGCATAA AGTTTAAACC TAAATCAAAG AAAAAGTCCC AATTTGTCGA CCAGAAAATT 5700
AACACAGTCT TGTCATGTTA TTTTTCAAAC GTTAGTTCTG AAGTTATGCC GCTCTCGCCA 5760
TTTTATATTC GTCACGAAGC CAAGCAGCTT GATATATATT TTAACAAATT CGGTTCAAAT 5820
GAGATTTTGT TAAGCATATG GGATACTGAT TTTTTCATGA CATCGCACCA GACAAAGGAG 5880
CAATACCTAA GGTTTTCATT TGGCGATATT GAAATTAAAG GAGGAATTTC TAGAGAAGGC 5940
TATTCGTTGA TAAACGTTGA CATCTCAATA TCTATGATTA AGTTAACCTT TTCGGAGCCG 6000
CGCCGTATTG TAAACAGTTT TTTACAAGAT GAAAAGCTTG CTTCTCAGGG TATCAATCTG 6060
TTATATTCCC TGAAGCCTTT ATTCTTTAGT TCAAATCTAC CAAAAAAAGA GAAGCAGGCA 6120
CCCTCGATAA TGATAAATTG GACATTAGAT ACTAGCATTA CTTATTTTGG TGTTCTTGTG 6180
CCAGTGGCTT CCACGTATTT CGTGTTTGAA TTACATATGC TGCTACTTTC TCTGACCAAT 6240
ACGAATAACG GTATGTTACC AGAAGAAACC AAGGTGACGG GACAGTTTTC CATCGAAAAC 6300
ATCCTATTTC TAATAAAGGA GCGGTCACTA CCCATTGGTC TTTCCAAATT ACTCGACTTT 6360
TCCATAAAAG TATCAACCCT ACAAAGAACG GTTGATACGG AGCAGTCATT CCAAGTGGAA 6420
AGTTCTCATT TCAGGGTCTG CTTATCTCCT GATTCTCTAT TAAGATTAAT GTGGGGCGCG 6480
CATAAATTGC TAGACTTGAG CCATTACTAT TCAAGACGCC ATGCCCCTAA TATTTGGAAC 6540
ACTAAGATGT TCACCGGTAA AAGTGATAAG TCAAAAGAAA TGCCCATAAA TTTCCGTTCA 6600
ATACACATCC TGTCCTATAA ATTTTGTATT GGGTGGATAT TCCAGTATGG AGCAGGCTCC 6660
AATCCTGGGT TAATGTTAGG TTATAACAGA TTGTTTTCAG CATATGAAAA GGATTTTGGG 6720
AAATTCACAG TTGTGGACGC TTTTTTCTCT GTTCCGAATG GTAATACCTC AAGCACTTTT 6780
TTCTCTGAAG GAAACGAGAA AGACAAATAT AATAGAAGTT TCTTGCCAAA CATGCAAATA 6840
TCCTACTGGT TCAAAAGATG TGGTGAGTTG AAAGATTGGT TTTTTAGATT TCATGGTGAA 6900
GCACTGGATG TAAACTTTGT CCCGTCATTC ATGGATGTCA TTGAGTCTAC TTTACAATCC 6960
ATGCGAGCAT TTCAAGAGCT GAAAAAGAAC ATTCTGGATG TGTCCGAGAG TTTGCGTGCG 7020
GAAAATGATA ATTCTTATGC TAGTACCAGT GTCGAAAGTG CTTCGAGTAG TTTGGCTCCC 7080
TTTCTCGATA ACATTAGATC TGTTAACTCA AATTTCAAGT ATGACGGTGG TGTATTTAGG 7140
GTTTACACGT ACGAAGATAT TGAAACCAAG AGTGAGCCAT CTTTTGAAAT AAAAAGTCCA 7200
GTAGTCACTA TAAACTGTAC ATATAAACAT GATGAAGATA AAGTTAAGCC ACATAAATTC 7260
AGAACATTAA TCACTGTCGA CCCAACGCAT AATACTTTGT ATGCAGGATG TGCTCCTTTA 7320
TTAATGGAAT TTTCTGAAAG TCTGCAAAAG ATGATAAAGA AACATAGCAC CGACGAAAAA 7380
CCAAACTTTA CAAAACCTTC TTCGCAGAAT GTTGATTATA AGCGACTTTT GGATCAATTT 7440
GATGTGGCTG TAAAACTAAC ATCAGCCAAG CAACAGTTAA GTTTGAGCTG TGAACCAAAA 7500
GCTAAGGTTC AGGCAGATGT TGGATTTGAA TCGTTTTTGT TCAGTATGGC TACCAATGAG 7560
TTCGACTCTG AACAGCCTTT GGAGTTTTCT TTAACTCTAG AACACACAAA AGCGTCCATT 7620
AAGCACATAT TTTCAAGAGA AGTAAGTACG TCCTTTGAAG TTGGTTTCAT GGACTTGACG 7680
CTTTTATTTA CACATCCTGA TGTAATCAGT ATGTATGGAA CGGGGTTGGT TTCTGATCTA 7740
AGCGTCTTCT TCAATGTAAA GCAGCTCCAG AACCTGTATT TATTCTTGGA CATATGGAGG 7800
TTCAGTAGCA TTTTACACAC ACGGCCAGTG CAAAGAACTG TTAATAAGGA AATTGAAATG 7860
AGTTGATTAA CATCAACCAA CTATGCCGAT GCAGGTACGG AAATACCCTG GTGCTTTACA 7920
TTAATTTTTA CAAATGTTAG CGGAGACGTT GATTTGGGTC CTTCTCTCGG TATGATTTCA 7980
TTAAGGACAC AAAGAACATG GCTGGCCACA GATCATTATA ACGAGAAGCG GCAGTTACTG 8040
CATGCTTTCA CTGACGGTAT TAGCTTGACA TCAGAAGGTA GACTGAGTGG TTTATTTGAA 8100
GTTGCGAATG CAAGTTGGTT ATCAGAAGTA AAATGGCCAC CTGAAAAAAG CAAAAATACT 8160
CATCCATTAG TTTCCACCTC CCTGAATATT GATGATATAG CGGTAAAGGC TGCTTTTGAT 8220
TATCATATGT TCTTAATCGG CACTATAAGT AACATACACT TCCATCTTCA TAATGAAAAG 8280
GATGCCAAGG GGGTTCTACC TGATTTGCTG CAGGTCTCTT TTTCATCAGA TGAAATTATC 8340
CTCAGCTCTA CTGCATTAGT TGTAGCAAAT ATACTGGATA TCTACAACAC CATTGTACGT 8400
ATGAGGCAGG ATAATAAAAT ATCGTATATG GAGACGTTGA GAGATTCCAA TCCTGGTGAA 8460
TCTAGGCAAC CAATATTATA CAAAGACATT TTAAGATCGC TGAAACTACT CAGAACTGAT 8520
CTCTCGGTGA ATATCTCCTC TTCAAAGGTC CAGATTTCGC CAATATCTTT ATTCGATGTG 8580
GAAGTGTTAG TAATAAGAAT TGAAAAAGTC TCTATACGTT CCGAAACACA TTCGGGGAAA 8640
AAATTAAAGA CAGATTTGCA ACTACAAGTT TTAGATGTTT CTGCAGCGCT TTCTACTTCC 8700
AAAGAAGaAT TAGATGAGGA AGTTGGAGCT TCCATTGCTA TTGATGATTA CATGCATTAT 8760
GCTTCCAAGA TTGTCGGTGG TACTATCATT GATATTCCAA AACTTGCTGT TCATATGACA 8820
ACTTTACAAG AAGAAAAGAC AAATAATTTA GAATATCTAT TTGCTTGCTC TTTTTCAGAC 8880
AAAATATCTG TAAGGTGGAA TCTAGGGCCT GTAGACTTCA TAAAGGAAAT GTGGACTACA 8940
CATGTCAAAG CACTGGCAGT TCGTCGATCC CAGGTAGCAA ATATTTCCTT TGGACAAACT 9000
GAGGAAGAAC TTGAAGAATC AATTAAAAAG GAAGAAGCCG CTTCAAAGTT TAATTATATT 9060

```
GCACTAGAAG AACCGCAGAT CGAAGTGCCT CAGATAAGAG ATCTGGGAGA CGCCACTCCA   9120
CCTATGGAAT GGTTTGGTGT CAATAGAAAA AAATTTCCGA AATTCACTCA CCAAACCGCA   9180
GTTATCCCCG TCCAAAAGCT TGTTTATCTT GCAGAAAAGC AGTATGTCAA GATACTAGAT   9240
GATACGCATT AATCTCGTTC CTAATTATAT TCAAAAGTCC TTGATACCAT ATTTCTGGTT   9300
TATGTTTTAT AGTTTTTNAA CTACGTGATA TTTGCATATA GTATAGATTT CCTACGCCCC   9360
ATATTTATGA ATCTTGCATT CGAATTTTGC TTCAAAAAAC TTTGTTTAAA TTGTATTTTG   9420
AGAGTTTGTT AAGATATACA TATACTTTCT GTTTTTGGTG CATTATCATT GACCTTTTTT   9480
TTTTTATTCC TAACTGCTGC CCAAAAGAAG AAATAAACAG GCGGGAATTT GGGTAGTATC   9540
TTTAAACTTC TTTGATAGCA TGATATTACA ATCAGCAAGT GCTCTTGAAT TGATTATTGT   9600
ACTAGCTAAT TCTGTTTAAG A                                             9621
```

(3) INFORMATIONS CONCERNANT LA SEQ ID No. 2:

(i) CARACTERISTIQUES DE LA SEQUENCE:

(A) LONGUEUR: 2496 acides aminés
(B) TYPE: acide aminé
(D) CONFIGURATION: linéaire

(ii) TYPE DE MOLECULE: protéine

(xi) DESCRIPTION DE LA SEQ ID No. 2:

```
Met Glu Ala Ile Ser Gln Leu Arg Gly Val Pro Leu Thr His Gln Lys
1               5                   10                  15
Asp Phe Ser Trp Val Phe Leu Val Asp Trp Ile Leu Thr Val Val Val
            20                  25                  30
Cys Leu Thr Met Ile Phe Tyr Met Gly Arg Ile Tyr Ala Tyr Leu Val
        35                  40                  45
Ser Phe Ile Leu Glu Trp Leu Leu Trp Lys Arg Ala Lys Ile Lys Ile
    50                  55                  60
Asn Val Glu Thr Leu Arg Val Ser Leu Leu Gly Gly Arg Ile His Phe
65                  70                  75                  80
Lys Asn Leu Ser Val Ile His Lys Asp Tyr Thr Ile Ser Val Leu Glu
                85                  90                  95
Gly Ser Leu Thr Trp Lys Tyr Trp Leu Leu Asn Cys Arg Lys Ala Glu
            100                 105                 110
Leu Ile Glu Asn Asp Lys Ser Ser Ser Gly Lys Lys Ala Lys Leu Pro
        115                 120                 125
Cys Lys Ile Ser Val Glu Cys Glu Gly Leu Glu Ile Phe Ile Tyr Asn
    130                 135                 140
Arg Thr Val Ala Tyr Asp Asn Val Ile Asn Leu Leu Ser Lys Asp Glu
145                 150                 155                 160
Arg Asp Lys Phe Glu Lys Tyr Leu Asn Glu His Ser Phe Pro Glu Pro
                165                 170                 175
Phe Ser Asp Gly Ser Ser Ala Asp Lys Leu Asp Glu Asp Leu Ser Glu
            180                 185                 190
Ser Ala Tyr Thr Thr Asn Ser Asp Ala Ser Ile Val Asn Asp Arg Asp
        195                 200                 205
Tyr Gln Glu Thr Asp Ile Gly Lys His Pro Lys Leu Leu Met Phe Leu
    210                 215                 220
Pro Ile Glu Leu Lys Phe Ser Arg Gly Ser Leu Leu Leu Gly Asn Lys
225                 230                 235                 240
Phe Thr Pro Ser Val Met Ile Leu Ser Tyr Glu Ser Gly Lys Gly Ile
                245                 250                 255
Ile Asp Val Leu Pro Pro Lys Glu Arg Leu Asp Leu Tyr Arg Asn Lys
            260                 265                 270
```

```
Thr Gln Met Glu Phe Lys Asn Phe Glu Ile Ser Ile Lys Gln Asn Ile
        275                 280                 285
Gly Tyr Asp Asp Ala Ile Gly Leu Lys Phe Lys Ile Asp Arg Gly Lys
    290                 295                 300
Val Ser Lys Leu Trp Lys Thr Phe Val Arg Val Phe Gln Ile Val Thr
305                 310                 315                 320
Lys Pro Val Val Pro Lys Lys Thr Lys Lys Ser Ala Gly Thr Ser Asp
                325                 330                 335
Asp Asn Phe Tyr His Lys Trp Lys Gly Leu Ser Leu Tyr Lys Ala Ser
            340                 345                 350
Ala Gly Asp Ala Lys Ala Ser Asp Leu Asp Asp Val Glu Phe Asp Leu
        355                 360                 365
Thr Asn His Glu Tyr Ala Lys Phe Thr Ser Ile Leu Lys Cys Pro Lys
    370                 375                 380
Val Thr Ile Ala Tyr Asp Val Asp Val Pro Gly Val Val Pro His Gly
385                 390                 395                 400
Ala His Pro Thr Ile Pro Asp Ile Asp Gly Pro Asp Val Gly Asn Asn
                405                 410                 415
Gly Ala Pro Pro Asp Phe Ala Leu Asp Val Gln Ile His Gly Gly Ser
            420                 425                 430
Ile Cys Tyr Gly Pro Trp Ala Gln Arg Gln Val Ser His Leu Gln Arg
        435                 440                 445
Val Leu Ser Pro Val Val Ser Arg Thr Ala Lys Pro Ile Lys Lys Leu
    450                 455                 460
Pro Pro Gly Ser Arg Arg Ile Tyr Thr Leu Phe Arg Met Asn Ile Ser
465                 470                 475                 480
Ile Met Glu Asp Thr Thr Trp Arg Ile Pro Thr Arg Glu Ser Ser Lys
                485                 490                 495
Asp Pro Glu Phe Leu Lys His Tyr Lys Glu Thr Asn Glu Glu Tyr Arg
            500                 505                 510
Pro Phe Gly Trp Met Asp Leu Arg Phe Cys Lys Asp Thr Tyr Ala Asn
        515                 520                 525
Phe Asn Ile Ser Val Cys Pro Thr Val Gln Gly Phe Gln Asn Asn Phe
    530                 535                 540
His Val His Phe Leu Glu Thr Glu Ile Arg Ser Ser Val Asn His Asp
545                 550                 555                 560
Ile Leu Leu Lys Ser Lys Val Phe Asp Ile Asp Gly Asp Ile Gly Tyr
                565                 570                 575
Pro Leu Gly Trp Asn Ser Lys Ala Ile Trp Ile Ile Asn Met Lys Ser
            580                 585                 590
Glu Gln Leu Glu Ala Phe Leu Leu Arg Glu His Ile Thr Leu Val Ala
        595                 600                 605
Asp Thr Leu Ser Asp Phe Ser Ala Gly Asp Pro Thr Pro Tyr Glu Leu
    610                 615                 620
Phe Arg Pro Phe Val Tyr Lys Val Asn Trp Glu Met Glu Gly Tyr Ser
625                 630                 635                 640
Ile Tyr Leu Asn Val Asn Asp His Asn Ile Val Asn Asn Pro Leu Asp
                645                 650                 655
Phe Asn Glu Asn Cys Tyr Leu Ser Leu His Gly Asp Lys Leu Ser Ile
            660                 665                 670
Asp Val Thr Val Pro Arg Glu Ser Ile Leu Gly Thr Tyr Thr Asp Met
        675                 680                 685
Ser Tyr Glu Ile Ser Thr Pro Met Phe Arg Met Met Leu Asn Thr Pro
    690                 695                 700
Pro Trp Asn Thr Leu Asn Glu Phe Met Lys His Lys Glu Val Gly Arg
705                 710                 715                 720
Ala Tyr Asp Phe Thr Ile Lys Gly Ser Tyr Leu Leu Tyr Ser Glu Leu
                725                 730                 735
```

Asp Ile Asp Asn Val Asp Thr Leu Val Ile Glu Cys Asn Ser Lys Ser
740 745 750
Thr Val Leu His Cys Tyr Gly Phe Val Met Arg Tyr Leu Thr Asn Val
755 760 765
Lys Met Asn Tyr Phe Gly Glu Phe Phe Asn Phe Val Thr Ser Glu Glu
770 775 780
Tyr Thr Gly Val Leu Gly Ala Arg Glu Val Gly Asp Val Thr Thr Lys
785 790 795 800
Ser Ser Val Ala Asp Leu Ala Ser Thr Val Asp Ser Gly Tyr Gln Asn
805 810 815
Ser Ser Leu Lys Asn Glu Ser Glu Asp Lys Gly Pro Met Lys Arg Ser
820 825 830
Asp Leu Lys Arg Thr Thr Asn Glu Thr Asp Ile Trp Phe Thr Phe Ser
835 840 845
Val Trp Asp Gly Ala Leu Ile Leu Pro Glu Thr Ile Tyr Ser Phe Asp
850 855 860
Pro Cys Ile Ala Leu His Phe Ala Glu Leu Val Val Asp Phe Arg Ser
865 870 875 880
Cys Asn Tyr Tyr Met Asp Ile Met Ala Val Leu Asn Gly Thr Ser Ile
885 890 895
Lys Arg His Val Ser Lys Gln Ile Asn Glu Val Phe Asp Phe Ile Arg
900 905 910
Arg Asn Asn Gly Ala Asp Glu Gln Glu His Gly Leu Leu Ser Asp Leu
915 920 925
Thr Ile His Gly His Arg Met Tyr Gly Leu Pro Pro Thr Glu Pro Thr
930 935 940
Tyr Phe Cys Gln Trp Asp Ile Asn Leu Gly Asp Leu Cys Ile Asp Ser
945 950 955 960
Asp Ile Glu Phe Ile Lys Gly Phe Phe Asn Ser Phe Tyr Lys Ile Gly
965 970 975
Phe Gly Tyr Asn Asp Leu Glu Asn Ile Leu Leu Tyr Asp Thr Glu Thr
980 985 990
Ile Asn Asp Met Thr Ser Leu Thr Val His Val Glu Lys Ile Arg Ile
995 1000 1005
Gly Leu Lys Asp Pro Val Met Lys Ser Gln Ser Val Ile Ser Ala Glu
1010 1015 1020
Ser Ile Leu Phe Thr Leu Ile Asp Phe Glu Asn Glu Lys Tyr Ser Gln
1025 1030 1035 1040
Arg Ile Asp Val Lys Ile Pro Lys Leu Thr Ile Ser Leu Asn Cys Val
1045 1050 1055
Met Gly Asp Gly Val Asp Thr Ser Phe Leu Lys Phe Glu Thr Lys Leu
1060 1065 1070
Arg Phe Thr Asn Phe Glu Gln Tyr Lys Asp Ile Asp Lys Lys Arg Ser
1075 1080 1085
Glu Gln Arg Arg Tyr Ile Thr Ile His Asp Ser Pro Tyr His Arg Cys
1090 1095 1100
Pro Phe Leu Leu Pro Leu Phe Tyr Gln Asp Ser Asp Thr Tyr Gln Asn
1105 1110 1115 1120
Leu Tyr Gly Ala Ile Ala Pro Ser Ser Ser Ile Pro Thr Leu Pro Leu
1125 1130 1135
Pro Thr Leu Pro Asp Thr Ile Asp Tyr Ile Ile Glu Asp Ile Val Gly
1140 1145 1150
Glu Tyr Ala Thr Leu Leu Glu Thr Thr Asn Pro Phe Lys Asn Ile Phe
1155 1160 1165
Ala Glu Thr Pro Ser Thr Met Glu Pro Ser Arg Ala Ser Phe Ser Glu
1170 1175 1180
Asp Asp Asn Glu Glu Gly Ala Asp Pro Ser Ser Phe Lys Pro Val Ala
1185 1190 1195 1200

Phe Thr Glu Asp Arg Asn His Glu Arg Asp Asn Tyr Val Val Asp Val
1205 1210 1215
Ser Tyr Ile Leu Leu Asp Val Asp Pro Leu Leu Phe Ile Phe Ala Lys
1220 1225 1230
Ser Leu Leu Glu Gln Leu Tyr Ser Glu Asn Met Val Gln Val Leu Asp
1235 1240 1245
Asp Ile Glu Ile Gly Ile Val Lys Arg Leu Ser Asn Leu Gln Glu Gly
1250 1255 1260
Ile Thr Ser Ile Ser Asn Ile Asp Ile His Ile Ala Tyr Leu Asn Leu
1265 1270 1275 1280
Ile Trp Gln Glu Thr Gly Glu Glu Gly Phe Glu Leu Tyr Leu Asp Arg
1285 1290 1295
Ile Asp Tyr Gln Met Ser Glu Lys Ser Leu Glu Lys Asn Arg Thr Asn
1300 1305 1310
Lys Leu Leu Glu Val Ala Ala Leu Ala Lys Val Lys Thr Val Arg Val
1315 1320 1325
Thr Val Asn Gln Lys Lys Asn Pro Asp Leu Ser Glu Asp Arg Pro Pro
1330 1335 1340
Ala Leu Ser Leu Gly Ile Glu Gly Phe Glu Val Trp Ser Ser Thr Glu
1345 1350 1355 1360
Asp Arg Gln Val Asn Ser Leu Asn Leu Thr Ser Ser Asp Ile Thr Ile
1365 1370 1375
Asp Glu Ser Gln Met Glu Trp Leu Phe Glu Tyr Cys Ser Asp Gln Gly
1380 1385 1390
Asn Leu Ile Gln Glu Val Cys Thr Ser Phe Asn Ser Ile Gln Asn Thr
1395 1400 1405
Arg Ser Asn Ser Lys Thr Glu Leu Ile Ser Lys Leu Thr Ala Ala Ser
1410 1415 1420
Glu Tyr Tyr Gln Ile Ser His Asp Pro Tyr Val Ile Thr Lys Pro Ala
1425 1430 1435 1440
Phe Ile Met Arg Leu Ser Lys Gly His Val Arg Glu Asn Arg Ser Trp
1445 1450 1455
Lys Ile Ile Thr Arg Leu Arg His Ile Leu Thr Tyr Leu Pro Asp Asp
1460 1465 1470
Trp Gln Ser Asn Ile Asp Glu Val Leu Lys Glu Lys Lys Tyr Thr Ser
1475 1480 1485
Ala Lys Asp Ala Lys Asn Ile Phe Met Ser Val Phe Ser Thr Trp Arg
1490 1495 1500
Asn Trp Glu Phe Ser Asp Val Ala Arg Ser Tyr Ile Tyr Gly Lys Leu
1505 1510 1515 1520
Phe Thr Ala Glu Asn Glu Lys His Lys Gln Asn Leu Ile Lys Lys Leu
1525 1530 1535
Leu Lys Cys Thr Met Gly Ser Phe Tyr Leu Thr Val Tyr Gly Glu Gly
1540 1545 1550
Tyr Glu Val Glu His Asn Phe Val Val Ala Asp Ala Asn Leu Val Val
1555 1560 1565
Asp Leu Thr Pro Pro Val Thr Ser Leu Pro Ser Asn Arg Glu Glu Thr
1570 1575 1580
Ile Glu Ile Thr Gly Arg Val Gly Ser Val Lys Gly Lys Phe Ser Asp
1585 1590 1595 1600
Arg Leu Leu Lys Leu Gln Asp Leu Ile Pro Leu Ile Ala Ala Val Gly
1605 1610 1615
Glu Asp Asp Lys Ser Asp Pro Lys Lys Glu Leu Ser Lys Gln Phe Lys
1620 1625 1630
Met Asn Thr Val Leu Leu Val Asp Lys Ser Glu Leu Gln Leu Val Met
1635 1640 1645
Asp Gln Thr Lys Leu Ile Ser Arg Thr Val Gly Gly Arg Val Ser Leu
1650 1655 1660

Leu Trp Glu Asn Leu Lys Asp Ser Thr Ser Gln Ala Gly Ser Leu Val
1665 1670 1675 1680
Ile Phe Ser Gln Lys Ser Glu Val Trp Leu Lys His Thr Ser Val Ile
1685 1690 1695
Leu Gly Glu Ala Gln Leu Arg Asp Phe Ser Ile Leu Ala Thr Thr Glu
1700 1705 1710
Ala Trp Ser His Lys Pro Thr Ile Leu Ile Asn Asn Gln Cys Ala Asp
1715 1720 1725
Leu His Phe Arg Ala Met Ser Ser Thr Glu Gln Leu Val Thr Ala Ile
1730 1735 1740
Thr Glu Ile Arg Glu Ser Leu Met Met Ile Lys Glu Arg Ile Lys Phe
1745 1750 1755 1760
Lys Pro Lys Ser Lys Lys Lys Ser Gln Phe Val Asp Gln Lys Ile Asn
1765 1770 1775
Thr Val Leu Ser Cys Tyr Phe Ser Asn Val Ser Ser Glu Val Met Pro
1780 1785 1790
Leu Ser Pro Phe Tyr Ile Arg His Glu Ala Lys Gln Leu Asp Ile Tyr
1795 1800 1805
Phe Asn Lys Phe Gly Ser Asn Glu Ile Leu Leu Ser Ile Trp Asp Thr
1810 1815 1820
Asp Phe Phe Met Thr Ser His Gln Thr Lys Glu Gln Tyr Leu Arg Phe
1825 1830 1835 1840
Ser Phe Gly Asp Ile Glu Ile Lys Gly Gly Ile Ser Arg Glu Gly Tyr
1845 1850 1855
Ser Leu Ile Asn Val Asp Ile Ser Ile Ser Met Ile Lys Leu Thr Phe
1860 1865 1870
Ser Glu Pro Arg Arg Ile Val Asn Ser Phe Leu Gln Asp Glu Lys Leu
1875 1880 1885
Ala Ser Gln Gly Ile Asn Leu Leu Tyr Ser Leu Lys Pro Leu Phe Phe
1890 1895 1900
Ser Ser Asn Leu Pro Lys Lys Glu Lys Gln Ala Pro Ser Ile Met Ile
1905 1910 1915 1920
Asn Trp Thr Leu Asp Thr Ser Ile Thr Tyr Phe Gly Val Leu Val Pro
1925 1930 1935
Val Ala Ser Thr Tyr Phe Val Phe Glu Leu His Met Leu Leu Leu Ser
1940 1945 1950
Leu Thr Asn Thr Asn Asn Gly Met Leu Pro Glu Glu Thr Lys Val Thr
1955 1960 1965
Gly Gln Phe Ser Ile Glu Asn Ile Leu Phe Leu Ile Lys Glu Arg Ser
1970 1975 1980
Leu Pro Ile Gly Leu Ser Lys Leu Leu Asp Phe Ser Ile Lys Val Ser
1985 1990 1995 2000
Thr Leu Gln Arg Thr Val Asp Thr Glu Gln Ser Phe Gln Val Glu Ser
2005 2010 2015
Ser His Phe Arg Val Cys Leu Ser Pro Asp Ser Leu Leu Arg Leu Met
2020 2025 2030
Trp Gly Ala His Lys Leu Leu Asp Leu Ser His Tyr Tyr Ser Arg Arg
2035 2040 2045
His Ala Pro Asn Ile Trp Asn Thr Lys Met Phe Thr Gly Lys Ser Asp
2050 2055 2060
Lys Ser Lys Glu Met Pro Ile Asn Phe Arg Ser Ile His Ile Leu Ser
2065 2070 2075 2080
Tyr Lys Phe Cys Ile Gly Trp Ile Phe Gln Tyr Gly Ala Gly Ser Asn
2085 2090 2095
Pro Gly Leu Met Leu Gly Tyr Asn Arg Leu Phe Ser Ala Tyr Glu Lys
2100 2105 2110
Asp Phe Gly Lys Phe Thr Val Val Asp Ala Phe Phe Ser Val Pro Asn
2115 2120 2125

Gly Asn Thr Ser Ser Thr Phe Phe Ser Glu Gly Asn Glu Lys Asp Lys
2130 2135 2140
Tyr Asn Arg Ser Phe Leu Pro Asn Met Gln Ile Ser Tyr Trp Phe Lys
2145 2150 2155 2160
Arg Cys Gly Glu Leu Lys Asp Trp Phe Phe Arg Phe His Gly Glu Ala
2165 2170 2175
Leu Asp Val Asn Phe Val Pro Ser Phe Met Asp Val Ile Glu Ser Thr
2180 2185 2190
Leu Gln Ser Met Arg Ala Phe Gln Glu Leu Lys Lys Asn Ile Leu Asp
2195 2200 2205
Val Ser Glu Ser Leu Arg Ala Glu Asn Asp Asn Ser Tyr Ala Ser Thr
2210 2215 2220
Ser Val Glu Ser Ala Ser Ser Ser Leu Ala Pro Phe Leu Asp Asn Ile
2225 2230 2235 2240
Arg Ser Val Asn Ser Asn Phe Lys Tyr Asp Gly Gly Val Phe Arg Val
2245 2250 2255
Tyr Thr Tyr Glu Asp Ile Glu Thr Lys Ser Glu Pro Ser Phe Glu Ile
2260 2265 2270
Lys Ser Pro Val Val Thr Ile Asn Cys Thr Tyr Lys His Asp Glu Asp
2275 2280 2285
Lys Val Lys Pro His Lys Phe Arg Thr Leu Ile Thr Val Asp Pro Thr
2290 2295 2300
His Asn Thr Leu Tyr Ala Gly Cys Ala Pro Leu Leu Met Glu Phe Ser
2305 2310 2315 2320
Glu Ser Leu Gln Lys Met Ile Lys Lys His Ser Thr Asp Glu Lys Pro
2325 2330 2335
Asn Phe Thr Lys Pro Ser Ser Gln Asn Val Asp Tyr Lys Arg Leu Leu
2340 2345 2350
Asp Gln Phe Asp Val Ala Val Lys Leu Thr Ser Ala Lys Gln Gln Leu
2355 2360 2365
Ser Leu Ser Cys Glu Pro Lys Ala Lys Val Gln Ala Asp Val Gly Phe
2370 2375 2380
Glu Ser Phe Leu Phe Ser Met Ala Thr Asn Glu Phe Asp Ser Glu Gln
2385 2390 2395 2400
Pro Leu Glu Phe Ser Leu Thr Leu Glu His Thr Lys Ala Ser Ile Lys
2405 2410 2415
His Ile Phe Ser Arg Glu Val Ser Thr Ser Phe Glu Val Gly Phe Met
2420 2425 2430
Asp Leu Thr Leu Leu Phe Thr His Pro Asp Val Ile Ser Met Tyr Gly
2435 2440 2445
Thr Gly Leu Val Ser Asp Leu Ser Val Phe Phe Asn Val Lys Gln Leu
2450 2455 2460
Gln Asn Leu Tyr Leu Phe Leu Asp Ile Trp Arg Phe Ser Ser Ile Leu
2465 2470 2475 2480
His Thr Arg Pro Val Gln Arg Thr Val Asn Lys Glu Ile Glu Met Ser
2485 2490 2495